Europäisches Patentamt

European Patent Office

Office européen des brevets

(19)

(11) Numéro de publication: **0 140 762**
**A1**

(12) # DEMANDE DE BREVET EUROPEEN

(21) Numéro de dépôt: **84401962.0**

(22) Date de dépôt: **02.10.84**

(51) Int. Cl.⁴: **C 12 N 15/00,** C 12 P 21/02,
A 61 K 39/205, C 07 K 15/14

(30) Priorité: **03.10.83 FR 8315716**
**27.03.84 FR 8404754**

(43) Date de publication de la demande: **08.05.85**
**Bulletin 85/19**

(84) Etats contractants désignés: **AT BE CH DE FR GB IT LI
LU NL SE**

(71) Demandeur: **TRANSGENE S.A., 95 rue Saint-Lazare,
F-75009 Paris (FR)**

(72) Inventeur: **Lathe, Richard, 8, quai des Bateliers,
F-67000 Strasbourg (FR)**
Inventeur: **Kieny, Marie Paule, 11, rue de Gascogne,
F-67100 Strasbourg (FR)**
Inventeur: **Lemoine, Yves, 4, rue des Alisiers,
F-67100 Strasbourg (FR)**
Inventeur: **Loison, Gérard, 1, rue de l'Aimant,
F-67000 Strasbourg (FR)**
Inventeur: **Aigle, Michel, 151, route Burkel,
F-67400 Illkirch-graffenstaden (FR)**
Inventeur: **Lecoco, Jean Pierre, 6, rue du Champ du Feu,
F-67116 Reichsteet (FR)**

(74) Mandataire: **Warcoin, Jacques et al, Cabinet
Régimbeau 26, avenue Kléber, F-75116 Paris (FR)**

(54) **Vecteurs d'expression d'une proteine antigenique de la rage dans les cellules eucaryotes et leur application à la préparation d'un vaccin.**

(57) La présente invention concerne des vecteurs d'expression d'une protéine antigénique de la rage dans les cellules euca-ryotes, caractérisés en ce qu'ils comportent au moins:
— une séquence d'ADN (1) codant pour ladite protéine antigénique de la rage, et
— les éléments d'expression de cette séquence dans une cellule eucaryote.
    Ces vecteurs peuvent être utilisés pour transformer des cellules eucaryotes afin de leur faire produire une glycopro-téine antigénique de la rage.

EP 0 140 762 A1

## VECTEURS D'EXPRESSION D'UNE PROTEINE ANTIGENIQUE DE LA RAGE DANS LES CELLULES EUCARYOTES ET LEUR APPLICATION A LA PREPARATION D'UN VACCIN

La présente invention concerne plus particulièrement l'élaboration d'un vaccin contre la rage.

La rage est une maladie, provoquée par le virus rabique, qui se présente sous forme d'une encéphalomyélite gravissime qui est presque toujours fatale en l'absence de traitement avant l'apparition des symptômes.

La rage est une zoonose. Tous les animaux à sang chaud, qu'ils soient mammifères ou oiseaux, sont réceptifs. Le principal vecteur est en général constitué par des mammifères mordeurs, qu'ils soient domestiques ou sauvages.

Les mesures de prévention efficaces ont permis de limiter les cas de rage humaine, qui se réduisent à quelques cas accidentels dans les pays fortement industrialisés.

Néanmoins, la rage reste encore une maladie très importante dans certains pays d'Amérique du Sud, en Afrique et en Asie.

L'arme essentielle de la lutte contre la rage pourrait être la prévention par la vaccination.

Les vaccins actuellement utilisés sont constitués par des virus inactivés.

Toutefois, pour être reproduits industriellement, ces virus doivent être cultivés sur des milieux tels que des cerveaux de souriceaux nouveau-nés, sur des embryons d'oiseaux, sur des cellules rénales animales ou sur des cellules diploïdes humaines, après quoi ils sont complètement inactivés par action de $\beta$-propiolactone par exemple.

L'utilisation de vaccins inactivés pose toujours le problème du risque d'une inactivation insuffisante. Enfin la nature particulière des milieux de culture conduit à de nombreuses difficultés au stade industriel qui font que le vaccin antirabique reste actuellement un vaccin très cher.

La Société TRANSGENE a déjà apporté une première solution à ce problème grâce à des vecteurs qui assurent l'expression de la protéine antigénique de la rage dans les bactéries tel que E. coli (voir en particulier le FR-A 82 08401).

Le but de la présente invention est la préparation d'une protéine antigénique vaccinante exempte de risque d'infection accidentelle qui, en outre, peut être préparée dans des conditions industrielles telles qu'elle assure un coût très faible du vaccin en n'utilisant plus des bactéries comme agents de production, mais des cellules eucaryotes. L'un des avantages potentiels des cellules eucaryotes en tant qu'élément d'expression est qu'elles sont susceptibles de fabriquer des antigènes de structure plus proche de celle de la rage et plus complètes que les bactéries.

Le virus de la rage est un <u>rhabdovirus</u>.

Ce rhabdovirus est constitué d'une nucléocapdite hélicoïdale contenant un ARN inclus dans une enveloppe lipoprotéinique ayant la forme d'un obus.

Ce virus comprend cinq protéines qui sont codées par le génome : G (glycoprotéine), N (nucléocapdite), M1 et M2 (matrice) et L (large, transcriptase). Comme la couverture membranaire provenant de l'hôte, le seul antigène efficace présent sur la surface est une glycoprotéine qui est une protéine transmembranaire de poids moléculaire d'environ 65 000 daltons qui possède une activité hémagglutinante.

La glycoprotéine purifiée est environ six fois plus active dans les essais d'hémagglutination que le virus intact.

Comme son nom l'indique, la glycoprotéine du virus de la rage est glycosylée, bien que le rôle de cette glycosylation soit inconnu.

La séquence d'ADN complémentaire du mARN de la glycoprotéine du virus de la rage a été décrite dans l'article de A. Anilionis et al., Nature 294; 275-278 (1981).

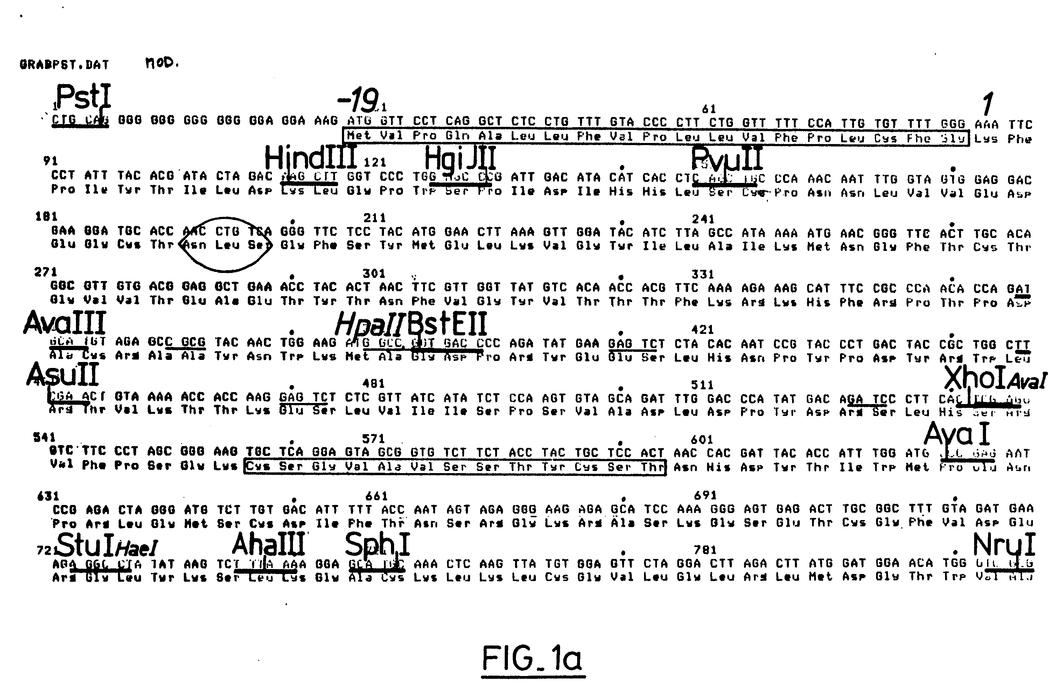

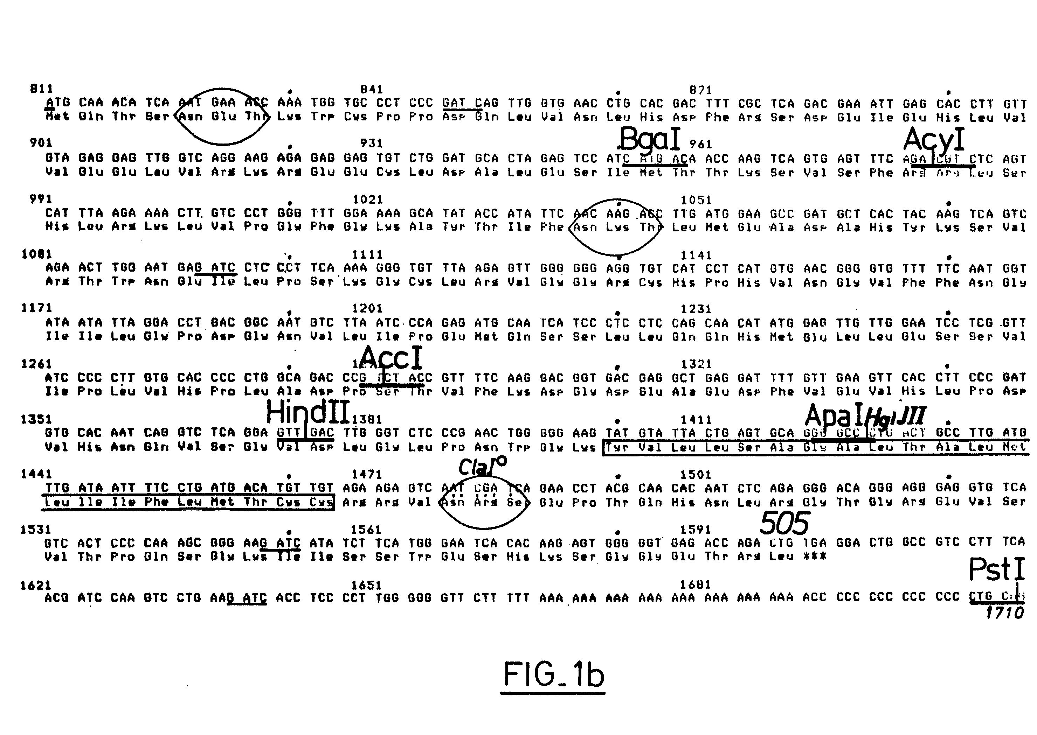

Ce gène a été utilisé dans le cadre des expérimentations qui seront décrites ci-après et est représenté schématiquement sur la figure 1 ci-annexée.

Le présente invention concerne un vecteur d'expression d'une protéine antigénique de la rage, dans les cellules eucaryotes, caractérisé en ce qu'il comporte au moins :

    . une séquence d'ADN (1) codant pour ladite protéine antigénique de la rage,

    . les éléments d'expression de cette séquence dans une cellule eucaryote.

Par séquence d'ADN (1) codant pour une protéine antigénique de la rage on entend désigner une séquence qui lorsqu'elle est exprimée dans les cellules eucaryotes conduit à une protéine antigénique de la rage.

Il peut s'agir de tout ou partie de la séquence d'ADN efficace codant pour la protéine antigénique de la rage ou bien d'une séquence d'ADN qui conduit, lorsqu'elle est exprimée, à une protéine ayant des propriétés imunologiques identiques ou très similaires à celle de la protéine antigénique de la rage.

Il est également intéressant d'utiliser l'ADN complet codant pour la protéine antigénique de la rage sous forme d'un fragment PstI - PstI. Dans un tel fragment on peut par exemple supprimer tout ou partie des G/C d'extrémité sans nuire à la qualité de la glycoprotéine exprimée.

Les éléments d'expression de l'ADN(1) varient selon la nature des cellules eucaryotes hôtes. On peut distinguer deux cas qui présentent actuellement un intérêt industriel : les cellules animales en particulier les cellules de mammifères, et les levures, bien que les autres types de cellules eucaryotes ne soient pas à écarter à priori.

Expression dans les cellules animales

Dans le cas où les cellules hôtes sont des cellules animales les éléments d'expression seront en général prélevés sur un virus par exemple des virus de la famille des Papovaviridiae tel que SV40 (virus simien 40, polyomavirus) ou le BPV (virus du papillome bovin, papillomavirus). Mais on peut utiliser d'autres virus tels que des adenovirus. L'un des avantages du virus SV40
est que sa réplication dans les cellules hôtes conduit
à une amplification considérable, plusieurs milliers
de copies.

Les éléments d'expression des vecteurs selon
l'invention comprendront au moins le promoteur d'un virus
qui peut être l'un des promoteurs du virus vecteur,
ainsi dans SV40 on peut utiliser le promoteur des gènes
tardifs, mais ce promoteur peut provenir d'un virus différent ainsi dans le BPV on utilisera ci-après le promoteur du gène de la thymidine kinase du virus Herpes Simplex.

Afin d'améliorer l'expression, les vecteurs
selon l'invention peuvent comprendre également des introns
et des sites de polyadémylation situés dans cet ordre à
l'extrémité de la séquence d'ADN (1). Ces éléments peuvent
là aussi provenir du virus vecteur ou bien être exogènes.
Ainsi dans le vecteur BPV les sites de polyadénylation
seront les sites précoces ou tardifs de SV40. Parmi les
introns utilisables il faut citer l'intron I du gène de la
$\beta$ globine de lapin, bien que cela ne soit nullement limitatif.

Enfin un vecteur d'expression autonome doit
être capable de se répliquer dans les cellules hôtes,
le vecteur devra donc comporter une origine de réplication, par exemple origine de réplication de SV40 ou de
BPV. En outre, le vecteur devra éventuellement être capable d'exprimer les protéines nécessaires à son maintient
et à sa réplication dans lesdites cellules comme l'antigène T pour SV40.

Dans certains cas les vecteurs comportant
l'ensemble de ces éléments peuvent devenir trop importants
pour être utilisables, dans ce cas certains des éléments
nécessaires au maintient et à la réplication du vecteur
peuvent être apportés par l'intermédiaire d'un autre vecteur ou bien être produits par les cellules elles-mêmes, en permettant de supprimer les parties correspondantes dans le vecteur.

Ainsi les cellules COS qui portent un génome de SV40 intégré et qui produisent l'antigène T nécessaire à la réplication de SV40, sont capables d'assurer la réplication d'un vecteur SV40 qui comporte une origine de réplication mais qui ne comporte pas les fonctions précoces de SV40.

Dans l'exemple qui sera décrit ci-après, la complémentation du vecteur d'expression proprement-dit (c'est-à-dire le vecteur portant la séquence d'ADN(1) sera assurée par un autre vecteur dit "vecteur de complément" assurant l'expression des éléments manquants au vecteur d'expression.

Dans certains cas il peut être intéressant d'introduire dans le vecteur d'expression un marqueur, gène de sélection positive ou négative dans les cellules hôtes ainsi par exemple un gène de résistance à un composé chimique particulier. Dans le cas de la construction BPV ce gène est le gène codant pour la dhfr (dihydrofolate-réductase) qui confère aux cellules la résistance au méthotrexate.

Ce gène de sélection permet la manipulation des vecteurs dans les cellules hôtes et peut, dans certains cas, assurer leur maintien.

Expression dans les levures.

Dans le cas où les cellules hôtes sont des levures, les éléments d'expression sont essentiellement constitués d'un promoteur et éventuellement d'un terminateur. Ces élément sont de préférence, bien que non nécessairement, des promoteurs et des terminateurs de gènes de la levure hôte. Ainsi lorsque la cellule hôte est Saccharomyces cereviseae, on pourra utiliser le promoteur et le terminateur du gène URA3 ou du gène PGK (phosphoglycerate kinase).

Comme dans le cas précédent le vecteur d'expression autonome doit être capable de se répliquer dans les levures, pour se faire il comportera une origine de réplication de levure telle que l'origine de réplication du plasmide $2\mu$, mais il est possible d'utiliser d'autres origines de réplication par exemple des origines de réplication d'origines chromosomiques.

Les vecteurs selon l'invention comporteront également de préférence un gène de sélection par exemple le gène LEU2 ou URA3 qui complémente une souche leu⁻ ou ura⁻.

Les vecteurs selon l'invention peuvent se présenter sous forme de vecteur autonome, c'est-à-dire sous forme de plasmide ou bien être intégrés dans les chromosomes des cellules eucaryotes, tant levures que cellules de mammifères dans ce cas il est possible d'intégrer le fragment codant pour l'ADN(1), par exemple le fragment promoteur/gène/terminateur en un ou plusieurs exemplaires dans les chromosomes (Weawer & coll. 1981).

De façon générale, les vecteurs selon l'invention comporteront en outre des éléments permettant leur maintien dans des bactéries et notamment comporteront une origine de réplication fonctionnelle dans E. coli (ori-eco) et un gène de sélection par exemple un gène de résistance à un antibiotique par exemple l'Ampicilline (Amp$^r$).

Ces éléments sont intéressants lors de la construction des vecteurs, lorsqu'on utilise comme

cellules hôtes des bactéries ; lors de l'expression de l'ADN(1) dans les cellules eucaryotes ces éléments pourront faire défaut.

Les vecteurs ainsi obtenus sont utilisés pour transformer ou transfecter les cellules eucaryotes correspondantes selon des processus connus.

Ainsi pour les levures on peut utiliser la technique de transfection décrite par H. Ito & coll 1983. Dans les exemples la souche utilisée est un ségregant particulier de Saccharomyces cereviseae (TG Y 1 sp1), mais d'autres souches de la même espèce ou d'autres espèces (Schizosaccharomyces pombes, S. uvanum) peuvent être également des hôtes convenables.

Dans le cas des cellules de mammifères la transfection peut être effectuée par exemple par la méthode décrite par Wigler et al 1978 pour les ADN cellulaires, par des techniques de fusion de protoplastes ou de microinjections, d'autres techniques sont connues lorsque le vecteur est sous forme virale. Les cellules hôtes de mammifères peuvent être variées mais adaptées au vecteur mis en oeuvre.

Les cellules transformées ou transfectées sont cultivées sur un milieu approprié assurant leur croissance.

Après culture, les cellules sont récoltées et la protéine antigénique de la rage peut être isolée par des techniques connues dans le domaine de purification des protéines et des antigènes.

Les antigènes ainsi préparés sont utiles tant pour le traitement et la prévention de la rage comme les antigènes préparés par les procédés "classiques", mais peuvent également être utilisés dans les diagnostiques in vitro de cette maladie.

En outre, la présente invention concerne une forme particulière de la protéine antigénique de la rage. En effet, à travers tout le règne vivant, la N-glycosylation (au niveau d'un résidu asparagine) d'une protéine donnée dépendra au minimum de trois facteurs relativement bien établis :

- l'existence au niveau du résidu asparagine envisagé de la structure primaire Asp-X-Ser ou Asp-X-Ther où X peut représenter tout acide aminé avec toutefois des restrictions pour l'aspartate et la proline ;

- l'accessibilité de ce résidu asparagine à la glycosyl transférase nécessite l'existence au niveau de cette séquence d'une structure secondaire donnée, bien souvent de type hélice $\beta$ ;

- le transit de cette protéine dans le réticulum endoplasmique où se trouvent localisés les enzymes de biosynthèse et de transfert (sur le résidu asparagine accepteur de la protéine) du précurseur universel, $(glc)_3$ $(man)_9$ $(glcNAc)_2$-P-P-dolichyl.

Cette dernière condition peut, en première analyse, être réalisée lorsque la protéine donnée est synthétisée sous forme d'un précurseur pourvu d'un "peptide signal" situé en général du côté aminoterminal de la protéine mature. En effet, la synthèse de ce peptide signal au niveau du complexe cytoplasmique de traduction permettrait à celui-ci, par un mécanisme moléculaire encore obscur, de migrer vers la membrane externe du réticulum endoplasmique. La synthèse de polypeptide se poursuivrait ensuite simultanément à la translocation de la chaîne en cours d'élongation à travers la membrane du réticulum endoplasmique. En couplage intime avec cette translocation, se produit dans le réticulum le transfert du précurseur universel $(glc)_3$ $(man)_9$ $(glcNAc)_2$-P-P-dolichyl sur les résidus asparagines potentiellement accepteurs, c'est-à-dire répondant au minimum aux conditions précitées.

La glycoprotéine de la rage, lors de l'infection de cellules par le virus de la rage, est synthétisée sous forme d'un précurseur comprenant une séquence signal amino terminale de 19 résidus d'acides aminés, de nature essentiellement hydrophobe.

Au niveau de la protéine mature, deux résidus asparagines (204 et 319), correspondent à deux des trois séquences concensus Asp X Ser/Thr existant dans la séquence primaire sont effectivement glycosylés.

Dans le vecteur d'expression dans la levure, décrit précédemment, le cDNA correspondant à la glycoprotéine de la rage contient l'information nécessaire à la synthèse du précurseur pourvu de la séquence signal.

De par sa qualité d'eucaryote, la levure est un hôte de choix pour obtenir des modifications post-traductionnelles spécifiques, à savoir, par exemple, la N glycosylation de la glycoprotéine rabique. Il n'est pas déraisonnable d'envisager que le précurseur de la glycoprotéine en voie de synthèse dans la levure soit dirigé vers le réticulum endoplasmique où se trouvent localisés les enzymes de synthèse et de transfert du précurseur $(glc_2)$ $(man)_9$ $(glcNAc)_2$-P-Pdolichyl. Si tel est le cas, on peut s'attendre à une N-glycosylation constituée exclusivement de résidus N acétylglucosamines et de résidus mannoses (éventuellement de résidus glucoses si aucune maturation n'a lieu ensuite).

C'est pourquoi la présente invention couvre une protéine antigénique de la rage caractérisée en ce qu'elle comporte une glycosylation de levure en particulier une glycosylation effectuée par une souche de S. cerevisiae.

De préférence, la glycosylation est effectuée au niveau des deux résidus asparagines 204 et 319.

Pour ce faire le vecteur mis en oeuvre pour transporter les levures est un vecteur tel que décrit précédemment, caractérisé en ce qu'il comporte dans la séquence ADN(I) tout ou partie de la séquence signal amino terminal codant pour les 19 résidus d'acides amines de ladite séquence signal.

Enfin, la présente invention concerne des vaccins ou des compositions curatives contenant à titre de principe actif, notamment, une protéine antigénique de la rage glycosylée décrite précédemment.

Les exemples ci-après sont destinés à mettre en évidence d'autres caractéristiques et avantages de la présente invention sans pour autant la limiter.

Sur les dessins ci-annexés :

la figure 1    représente la structure du gène codant
               pour la glycoprotéine de la rage.

la figure 2    représente la structure du virus SV40

la figure 3    schématise la synthèse de pg147

la figure 4    schématise la synthèse de pTG150

la figure 5    schématise la synthèse de pSVTG151

la figure 6    schématise la synthèse du vecteur de
               complémentation pTG152

la figure 7    schématise la synthèse de pTG155

la figure 8    explicite la technique permettant de
               supprimer les extrémités G/C en 5'
               d'un cDNA de la glycoprotéine dans
               pTG155

la figure 9    schématise la synthèse de SVTG171

la figure 10   schématise la synthèse de M13TG140

la figure 11   représente la structure simplifiée du BPV

la figure 12   représente la structure simplifiée
               du plasmide pMOP

la figure 13   représente le détail de la structure de
               pMOP au niveau du bloc d'expression du
               gène dhfr

la figure 14   représente la synthèse de pTG158 - SAL

la figure 15   représente la synthèse de pTG172

la figure 16   représente la synthèse de pTG856

la figure 17   représente la modification de l'extrémité
               du promoteur PGK

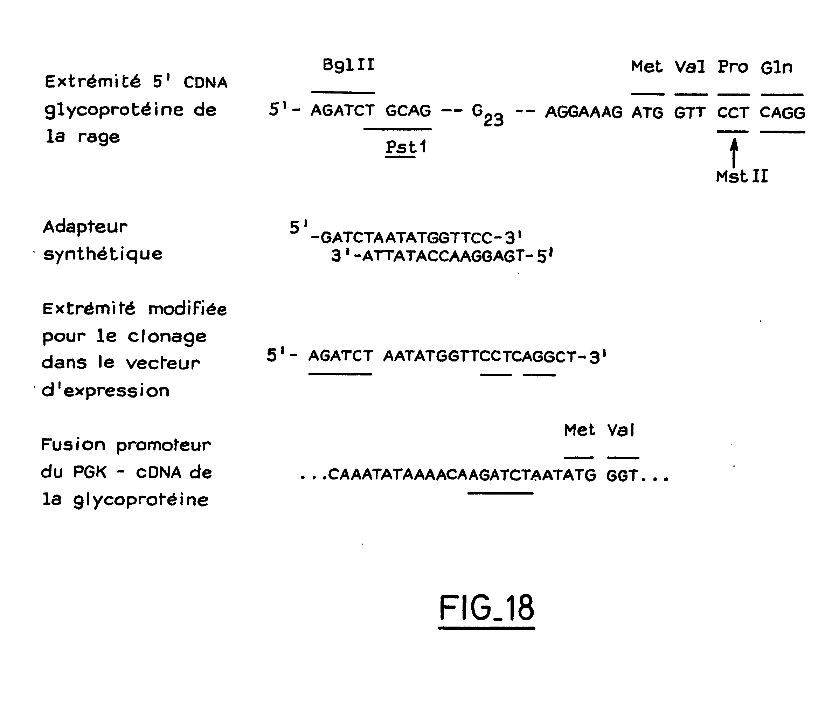

la figure 18   représente les éléments et la structure
               finale de la jonction entre le promoteur
               de levure et le gène de la rage

la figure 19   représente les immunoprécipités spécifiques du polypeptique de la glycoprotéine
               du virus de la rage dans les extraits de
               levure

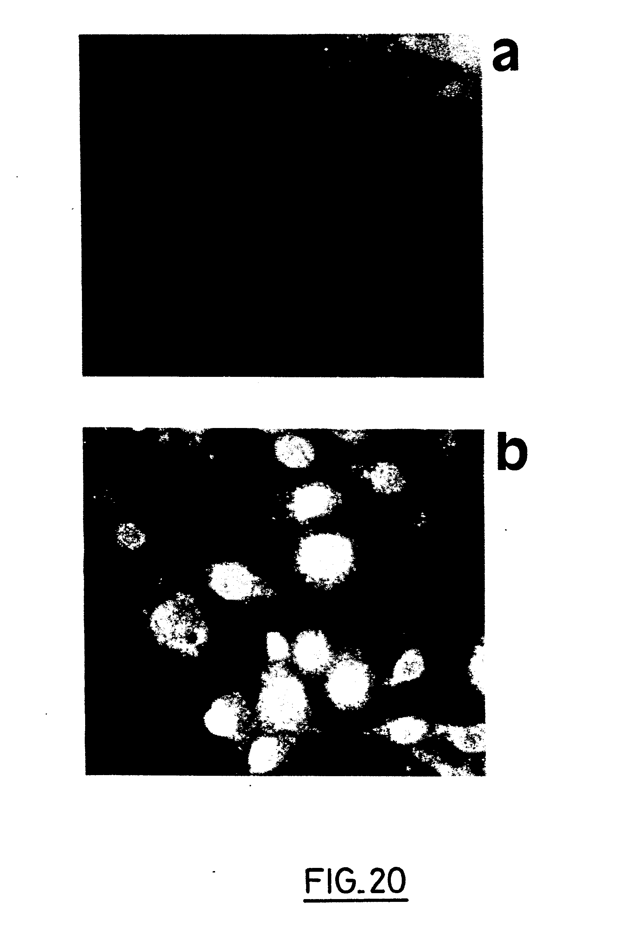

le figure 20   représente un cliché d'immunofluorescence
               obtenu à partir des extraits de cellule 3T3

0140762

Les matériels et méthodes suivants sont mis en oeuvre dans les exemples, sauf indications contraires :

### Souches et plasmides

La souche de bactérie utilisée est une souche E. coli 1106 (SupE hSdS met supF). TGY1sp1 est un segregeant obtenu par croisement de la souche GFR18 (MatΦ His3-11-15 Leu2-3-12) (FINK et coll.) avec la souche TGY10-1 (Mata Ura3-251-373-328). La souche TGY10-1 est un dérivé isogénique de la souche FL100 (ATCC 2283).

### Enzymes et produits chimiques

Tous les enzymes utilisés proviennent de Bethesda Research Laboratories, New England Biolab ou Borhienger Manhein.

Les oligonucléases utilisées proviennent soit de New England Biolab, Hana Biologics et Worthington Biochemical, ou sont synthétisés par les techniques Kohli et coll.

Tous les produits radioactifs ont été obtenus chez Radio Chemical Center Amerscham.

### Transformation

La transformation de E. coli 1106 par les plasmides est effectuée comme décrit par Dagert et Ehrlich 1979.

### Utilisation des enzymes de restriction

Les enzymes de restriction sont utilisées selon les conditions spécifiées par le fabricant. Les autres traitements enzymatiques sont effectués selon les techniques décrites par Maniatis et coll. (1982).

Après chaque traitement enzymatique, l'ADN est extrait avec le phénol, deux fois avec octanol - chloroforme (1 : 8) puis précipitation avec l'éthanol. Dans le cas de petits fragments (<100 bp) une triple extraction avec le diéthylether remplace la précipitation à l'éthanol.

L'analyse des produits de digestion est effectuée par électrophorèse sur gel d'agarose selon les techniques connues.

Hpa1 lié par l'intermédiaire d'un oligonucléotide "linker" Kpn1 à un fragment Hpa1-BamH1. Ainsi les sites de polyadénylation (A) pour la transcription immédiate et tardive de SV40 sont flanqués par un site Kpn1 et un site BamH1. Pour cloner ce plasmide sous forme d'un fragment EcoR1-Sal1, il est nécessaire d'écarter la partie entre les sites BamH1 et Sal1 (ce fragment étant déjà présent dans le virus vecteur), en outre, une répétition directe de ce fragment produirait une délétion par recombinaison homologue in vivo.

C'est pourquoi, pK14 est traité avec BamH1 et la nucléase S1 suivi par une digestion avec Sal1 et d'un traitement à l'ADN polymérase, afin de remplir les extensions 5' produites par le clivage avec Sal1. La fusion des deux extrémités par l'ADN ligase conduit à une reconstitution du site Sal1 dans pTG142.

Comme l'on désire insérer le cADN de la glycoprotéine, soit dans les sites EcoR1, soit dans le site Kpn1, il a été envisagé d'utiliser le site EcoR1 de pBR328, ce site est un site interne du gène plasmidique qui se trouve situé dans le gène de résistance au chloramphenicol. Ayant rencontré des problèmes de léthalité associée avec l'expression du gène de la glycoprotéine dans les bactéries on a transféré le fragment EcoR1-Sal1 dans les sites correspondants de pBR322 pour obtenir pTG143. On insère ensuite un linker BglII entre les deux sites EcoR1 et Kpn1 et on utilise la technologie des adaptateurs pour insérer le gène de la glycoprotéine.

L'insertion du gène de la glycoprotéine de la rage sous forme d'un fragment PstI - PstI provenant du plasmide pRG décrit par Anilianis 1981, dans le site BglII a été rendue possible en utilisant un adaptateur octamère 5' GATCTGCA 3' comme intermédiaire (Lathé et coll 1983).

0140762

L'insértion des "linkers" non phosphorylés a été effectuée de la façon suivante :

Les linkers sont repris dans du tampon TE (10mM Tris.HCl pH 7,5, 1 mM Na$_2$EDTA) à la concentration de 1 mg/ml et prehybridés par refroidissement de 65°C à 4°C. La ligaison est effectuée dans un tampon de concentration 30 mM NaCl, 30 mM Tris.HCl pH 7,5, 1mM spermidine, 0,25 mM ATP, 2 mM DTT, 0,2 mM Na$_2$EDTA et 0,1 mg/ml serum albumine bovine. En général, la ligaison (10 à 50 µl est effectuée avec un excès molaire du linker sur les extrémités d'ADN de 80 fois, et une concentration d'ADN cible de 0,1 µM. La ligase est utilisée à la concentration de 100 à 200 unités/ml et la ligaison est effectuée à 4°C pendant 16 heures. Les linkers excédentaires sont éliminés par précipitation à la spermine (Hoopes et Mc Clure, 1981). L'ADN est ensuite repris dans 10 mM Tris.HCl pH 7,5, 1 mM Na$_2$EDTA, puis dilué dans du tampon d'hybridation (100 mM Tris.HCl pH 7,5, 5 mM Na$_2$EDTA, 100 mM NaCl). La réhybridation est effectué comme la préhybidation des linkers, et des aliquots sont transformés directement dans E. coli. Le principe de la méthode est décrit par LATHE et Coll., 1983.

## Exemple 1 : Expression à l'aide du virus SV40

1 - L'expression du gène codant pour une protéine antigène de la rage (ci-après gène de la rage) dans les cellules de singe par l'intermédiaire de SV40 repose sur la complémentation entre deux vecteurs dont l'un porte le gène de la rage et une partie du génome de SV40 et l'autre porte la partie complémentaire du génome de SV40 ceci afin d'obtenir deux vecteur de dimensions réduites de l'ordre de 5200 bp (la taille de SV40 sans insertion) susceptibles de se complémenter et de s'encapsider.

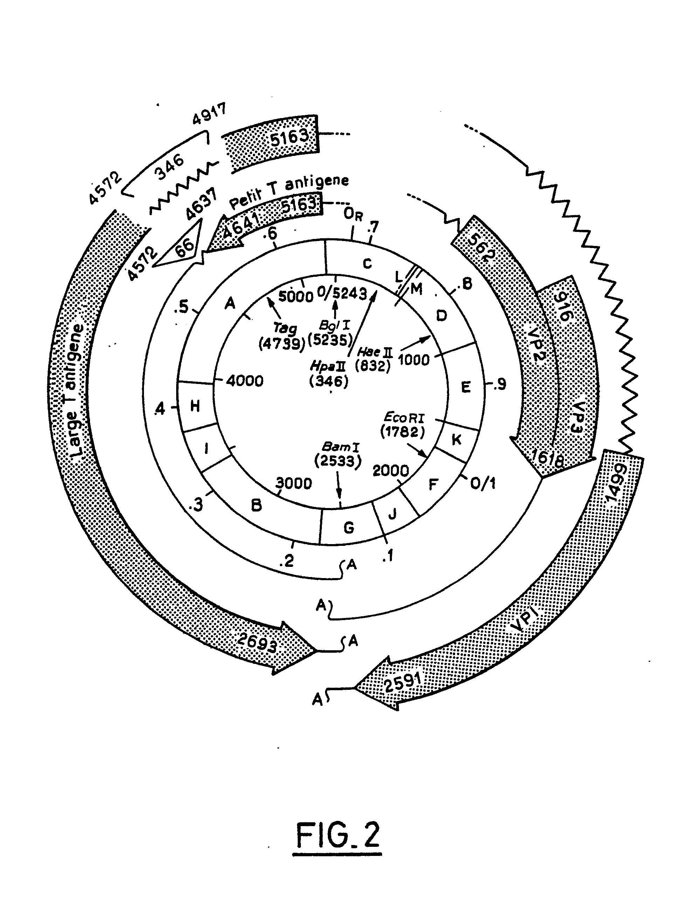

Le génome du virus simien schématisé sur la figure 2 d'après Griffin (1981) est constitué d'une molécule ADN circulaire à double brin comportant 5243 bp. Ce génome comporte pour ce qui nous intéresse une origine de réplication OR, une région précoce codant pour deux antigènes, les antigènes T et t et une région tardive comportant VP1, VP2 et VP3.

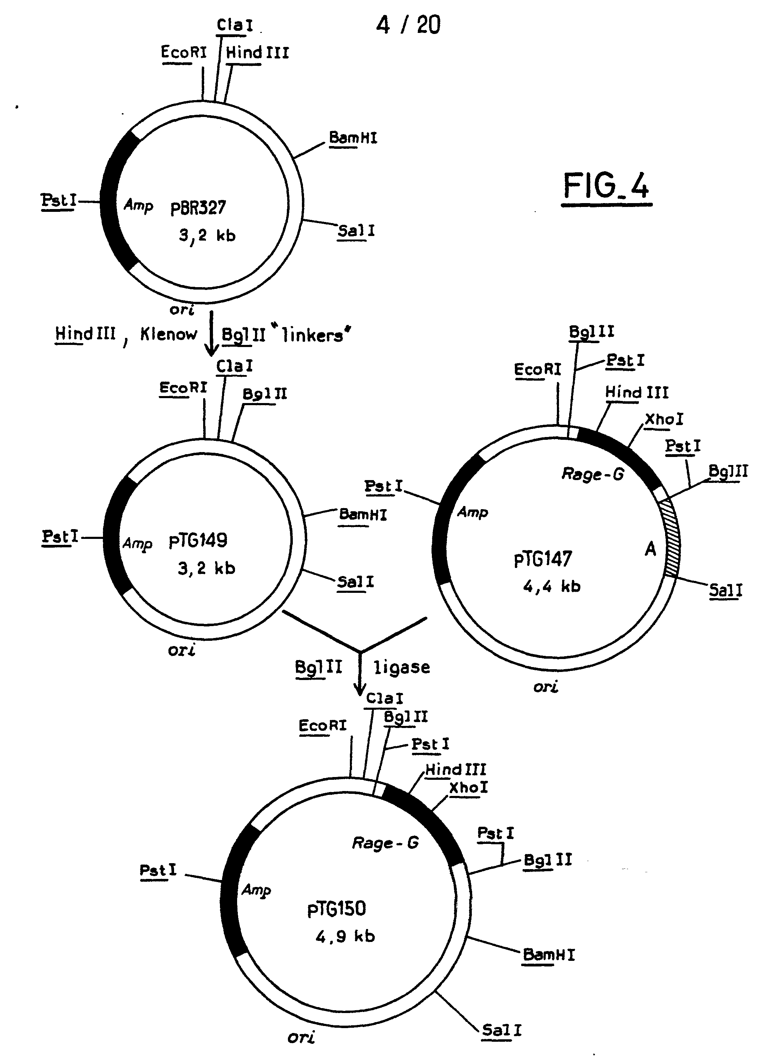

### Construction de pTG149 - figure 4

Le plasmide pBR327 (Soberon et coll 1980) est mis en digestion avec HindIII, les extrémités cohésives sont complémentées par traitement avec l'ADN polymérase Klenow, et le plasmide est lié avec un "linker" BglII octamère 5' dCAGATCTG-3'. Après réhybridation des extrémités de "linker" et transformation dans E. coli, on met en évidence les plasmides corrects par restriction avec BglII.

### Construction de pTG147

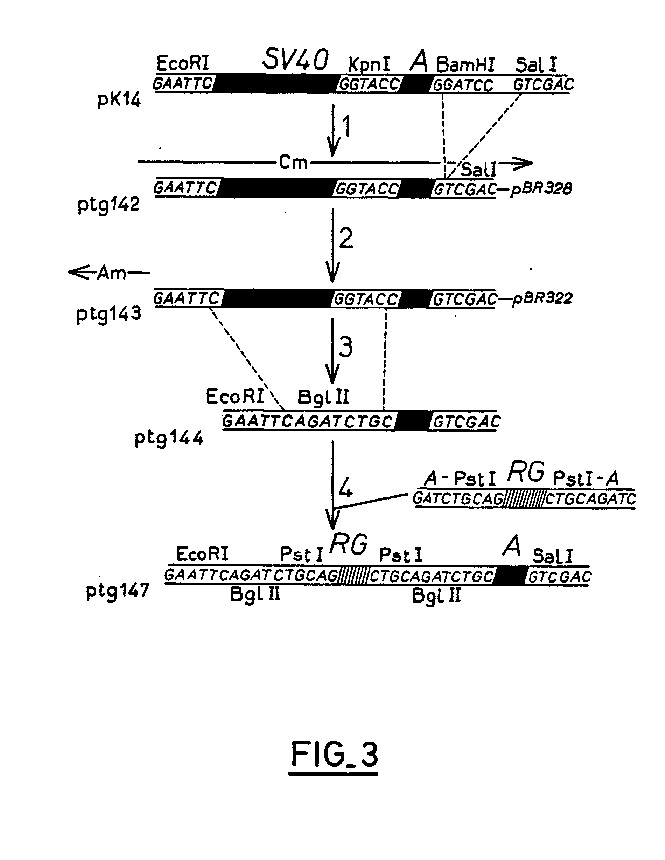

La construction de ce vecteur est schématisée sur la figure 3 ci- annexée.

Le plasmide pK 14 de départ comporte un fragment EcoR1-BamH1 provenant de SV40, inséré entre les sites EcoRI-BamH1 de pBR322.

Le fragment SV40 comprend un fragment EcoR1-

### Construction de pTG150 - figure 4

Les plasmides pTG147 et pTG149 sont digérés complètement avec Bg III, mélangés en quantité équimolaire, liés, et le mélange de ligation est utilisé pour transformer E. coli. Le plasmide pTG150 est identifié comme le plasmide comportant le cDNA complet de la glycoprotéine de la rage inséré dans le site BglII de pTG149.

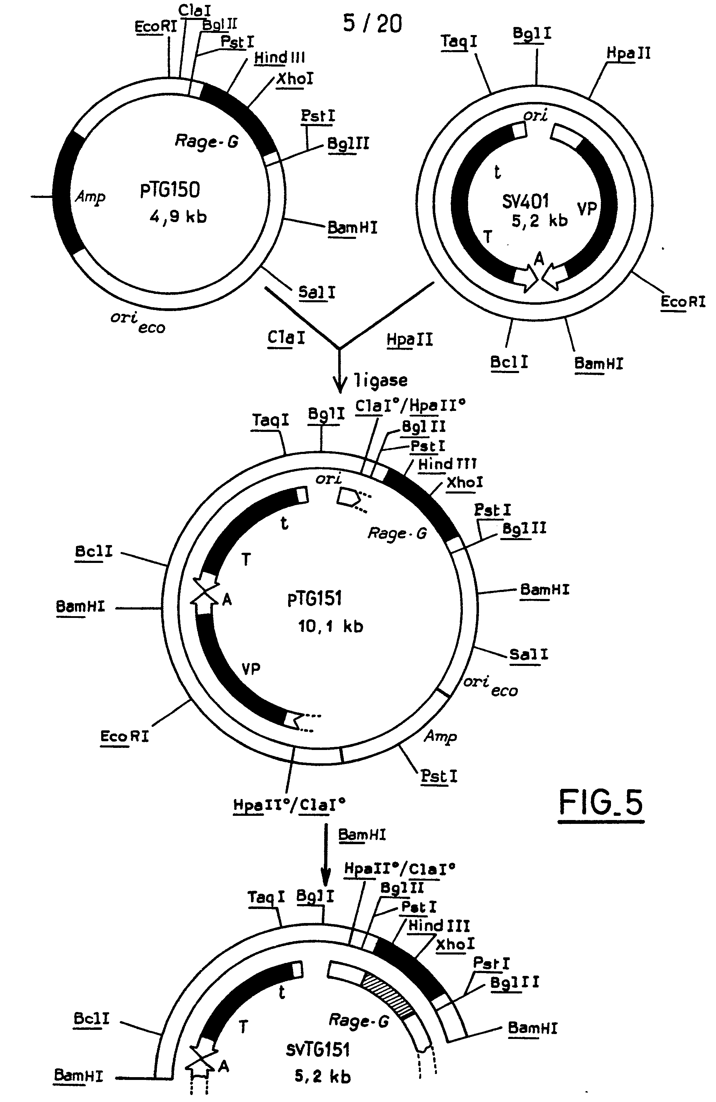

### Construction de pTG151 et de SVTG151 - figure 5

Le plasmide pTG150 est introduit entièrement sous forme d'un fragment de digestion ClaI dans le site de restriction HpaII du virus SV40 (dénomé ci-après SV401) en soumettant respectivement pTG150 à Cla1, SV40 à HpaII et en mettant les morceaux de restriction en présence de ligase.

On obtient ainsi le plasmide pTG151 de 10,1 kb.

Ce plasmide est trop grand pour pouvoir être introduit dans des cellules de mammifères. C'est pourquoi on l'utilise sous forme d'un fragment linéarisé par BamH1. En effet, le fragment BamH1 comporte, outre le gène de la glycoprotéine de la rage, la partie du génome de SV40 qui peut être complémentée par le plasmide pTG152 dont la construction va être explicitée ci-après.

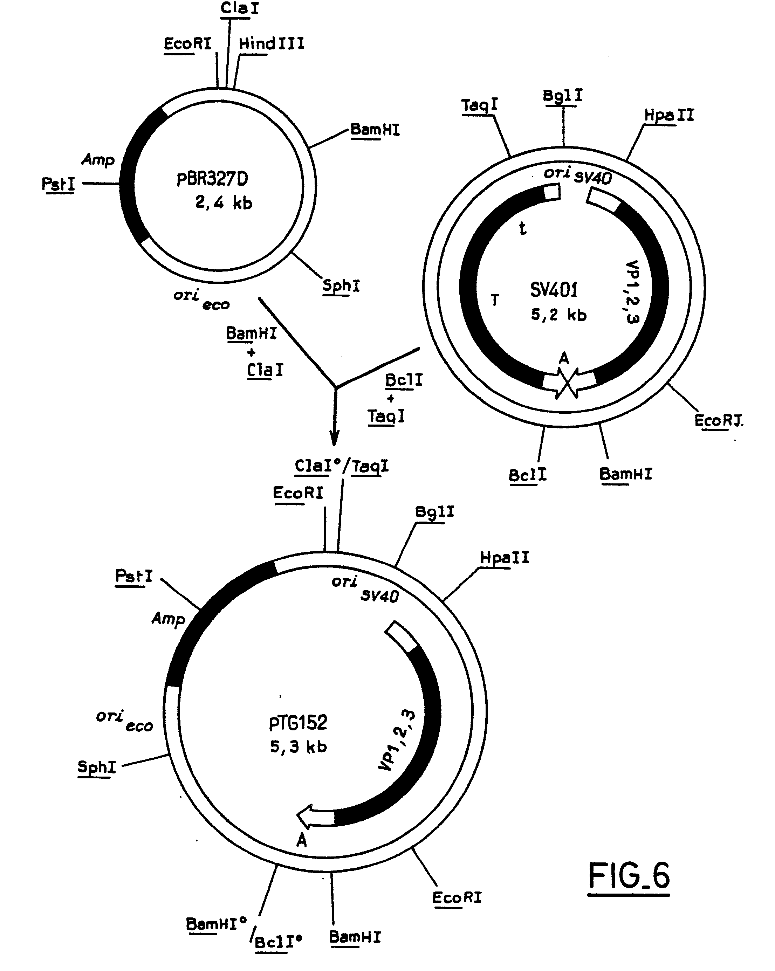

### Construction de pTG152 - figure 6

Le plasmide pTG152 est obtenu à partir de pBR327 D par digestion avev BamH1 et Cla1 afin d'isoler un fragment comportant une origine de réplication fonctionnelle dans E. coli (ori-eco) et le gène de résistance à l'ampicilline. Ce fragment est lié à un fragment obtenu par digestion de SV40 par Bcl1 et Taq1. On obtient ainsi le plasmide pTG152 de 5,3 kb qui comporte la partie complémentaire pour SV40 du plasmide SVTG151.

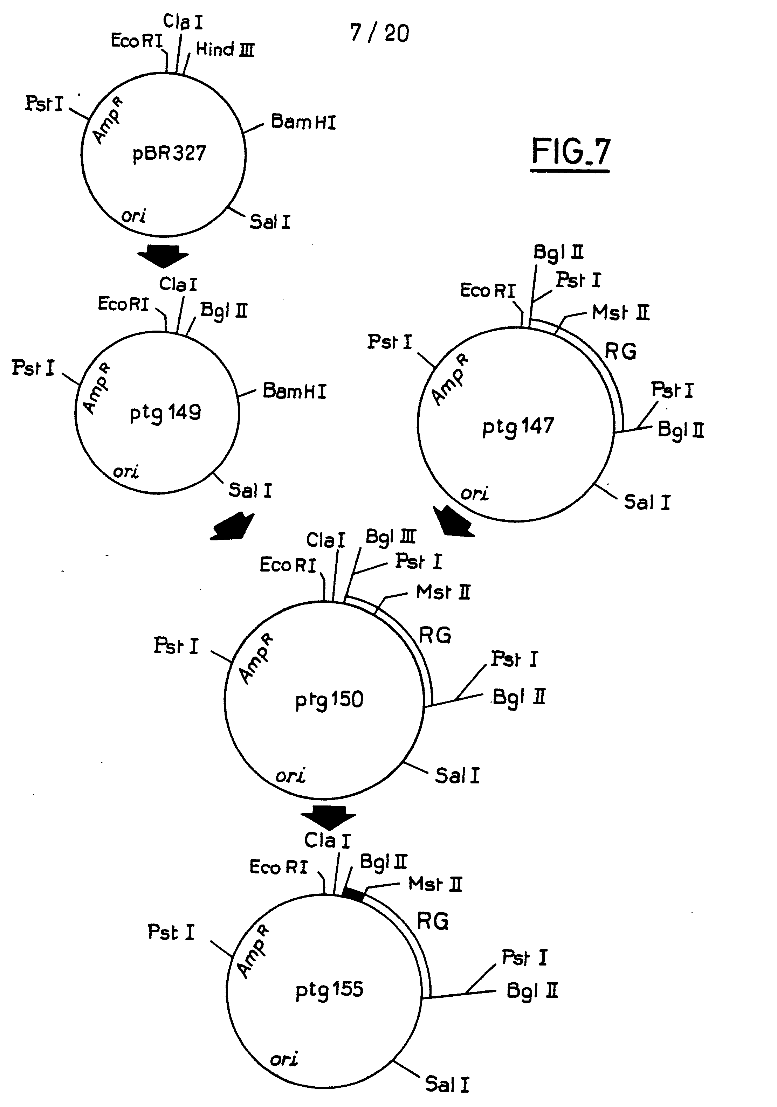

Exemple 2 : **Synthèse du vecteur SVTG160 - fig. 7**

**Synthèse du plasmide pTG155**

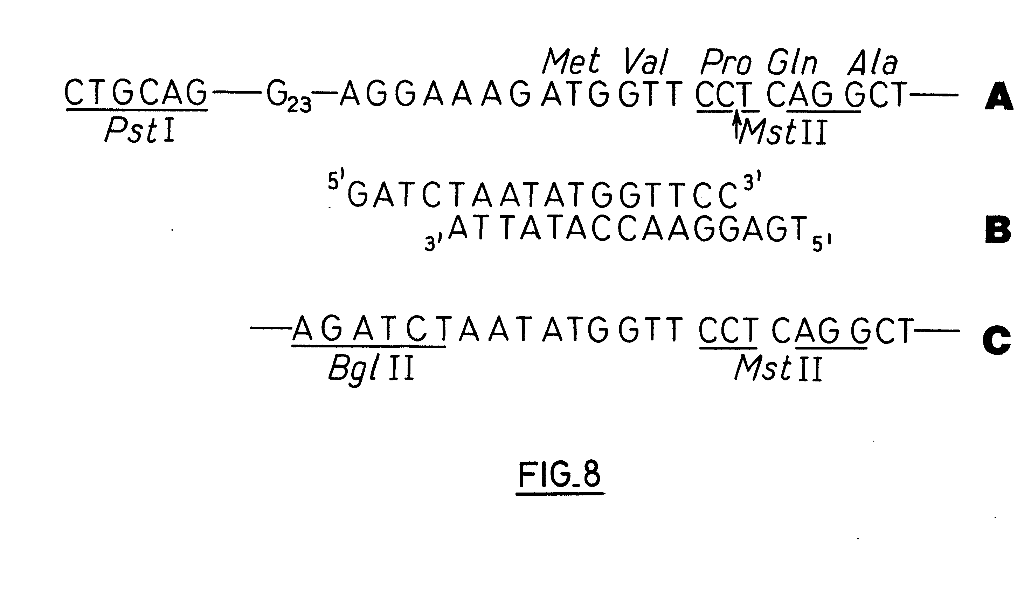

A partir du plasmide pTG150 on effectue une digestion partielle avec BglII puis ensuite une digestion complète avec MstII. Le plasmide est recircularisé par ligation en présence d'un adaptateur double brin synthétique dont la formule est donnée en B fig. 8, afin de fusionner les sites BglII et MstII pour déléter le polymère G/C à l'extrémité 5' de l'élément cDNA. Ce plasmide pTG155 contient un site d'initiation de la traduction reconstituée dans lequel le nucléotide adenine est situé en position -3 pour être conforme à la séquence généralement admise pour l'initiation de la traduction dans les cellules eucaryotes.

Ce plasmide peut être traité comme pTG150 pour donner le vecteur SVTG160.

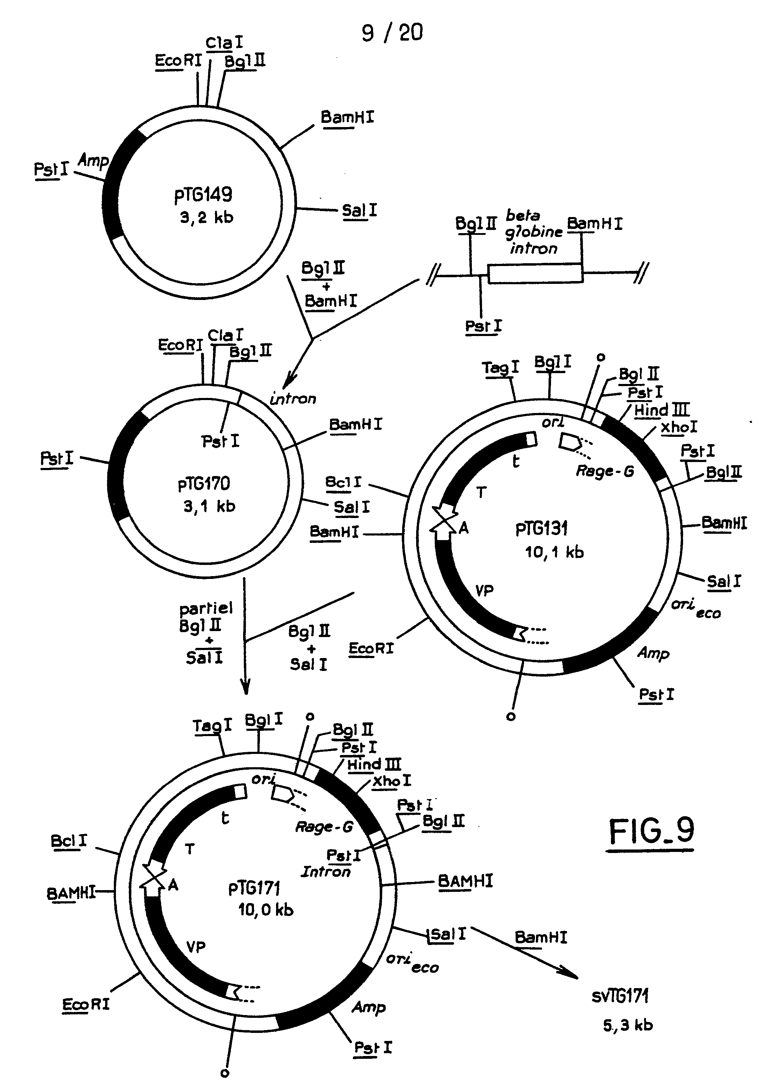

Exemple 3 : **Synthèse du vecteur SVTG171 - Fig. 9**

Ce vecteur se différencie des précédents en ce qu'il comporte un intron à la suite du gène de la rage.

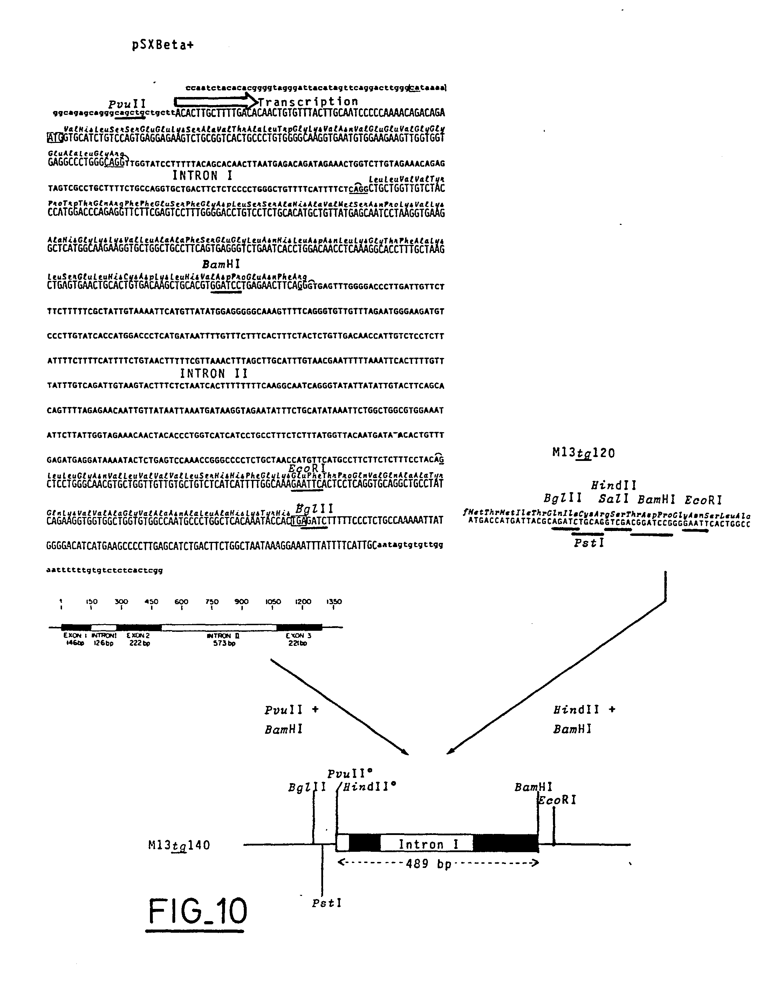

Cet intron est l'intron I provenant de pSXBETA[+] (β globine de lapin). Cet intron est prélevé sous forme d'un fragment PvuII/BamH1 et inséré dans un phase M13 en l'occurence M13 TG120 entre les sites HindII et BamH1. On obtient ainsi le phage M13 TG140 (fig.10) qui comporte l'intron I, lequel est prélevé sous forme d'un fragment BglII/BamH1 pour être inséré entre les sites correspondants de pTG149 (voir ci-dessus).

On obtient ainsi le plasmide pTG170.

Par digestion de pTG170 et de pTG151 (voir ci-dessus) par BglII et SalI et mise en présence des fragments de digestion on obtient le plasmide pTG171 qui, comme précédemment, est linéarisé par BamHI afin de fournir le vecteur SVTG171.

On peut dans des conditions analogues et en utilisant pTG160 obtenir un plasmide comportant un intron.

Les différents plasmides SVTG171, SVTG151 et SVTG160 complémentés par pTG152 dans les cellules de mammifères, permettent de produire une protéine antigénique de la rage.

Exemple 4 : Préparation d'un vecteur à partir du PVB.

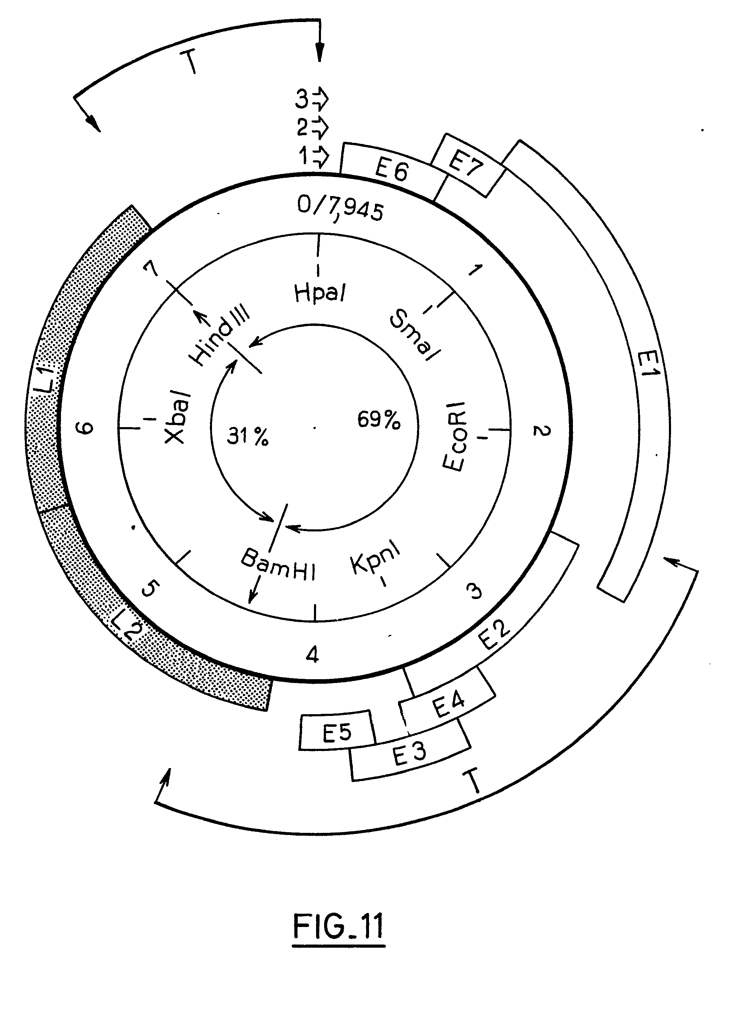

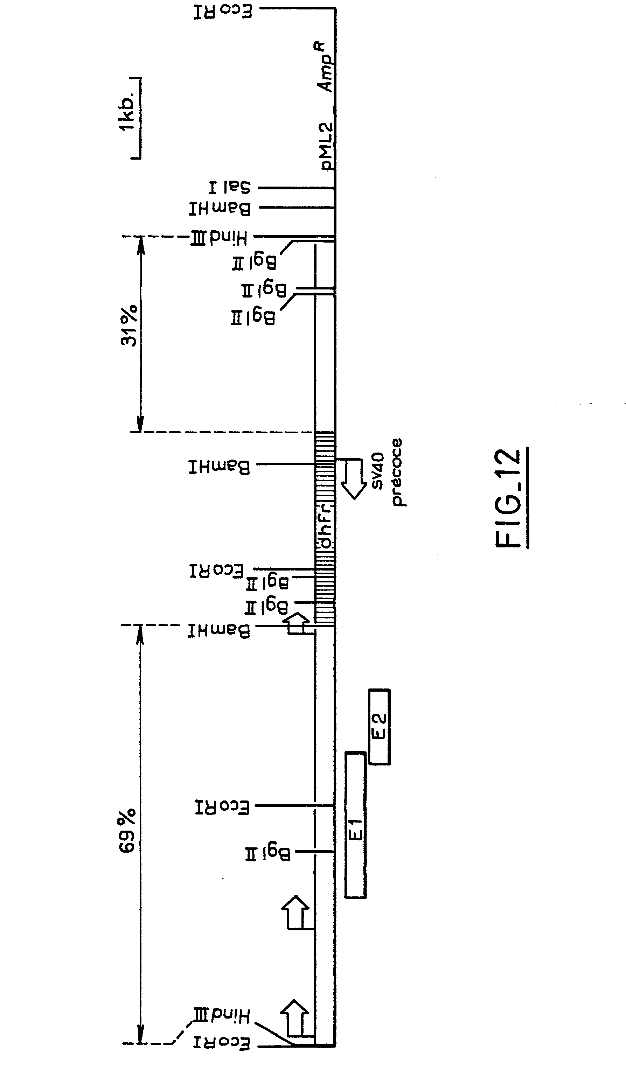

1) Le plasmide pMOP - figures 11 - 12

Le virus BPV se divise en deux parties limitées par deux sites uniques de restriction HindIII et BamH1 qui délimite deux fragments dit respectivement de "69 %" et de "31 %". (figure 11 d'après Chen et coll. 1982).

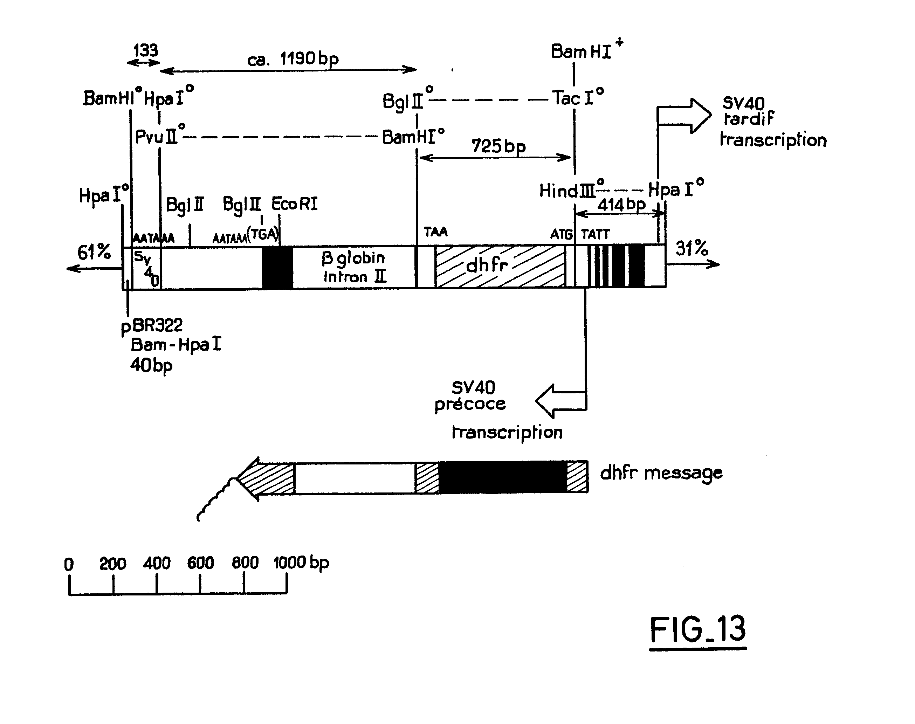

Le vecteur dérivé du BPV mis en oeuvre dans cet exemple est dénommé pMOP. Il est obtenu par introduction dans le site BamH1 du BPV du marqueur dhfr codant pour la dihydrofolate réductase sous le contrôle du promoteur précoce de SV40 (voir figure 13).

Ce vecteur pMOP comporte en outre un fragment de pBR322 portant le gène de résistance à l'ampicilline qui provient du plasmide pML2 décrit par Lusky et Botchan (1981). La figure 13 représente le détail de la structure de pMOP entre les sites BamH1, ces éléments ne constituent pas des éléments essentiels dans le cadre de la présente invention puisqu'ils sont destinés à assurer l'expression du gène dhfr.

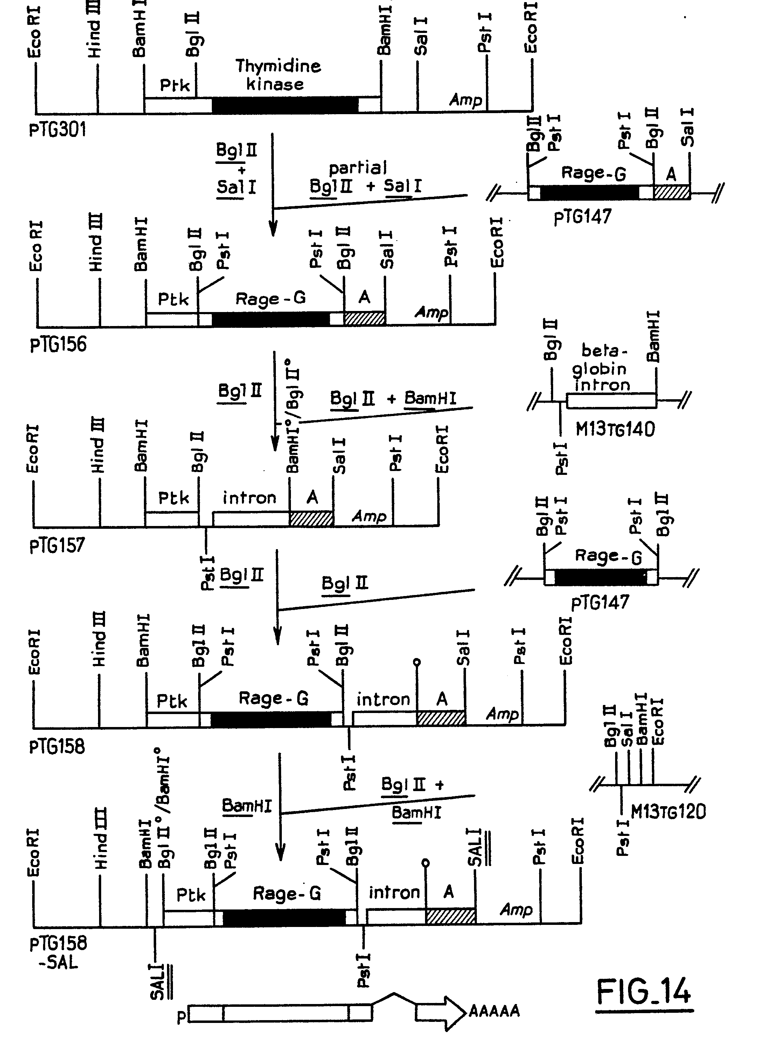

2) Le plasmide pTG158SAL - figure 14

Le gène codant pour la protéine antigénique de la rage provient du plasmide pTG147 dont la construction a été explicitée précédemment.

Ce gène est prélevé sous forme d'un fragment BglII/SalI par digestion partielle de pTG147 pour être introduit entre les sites correspondants du plasmide pTG301 traité par BglII/SalI. Ce plasmide pTG301 comporte le gène de la thymidine kinase (tk) et son promoteur (ptk), il est obtenu par insertion dans le site BamH1 de pBR322 d'un fragment BamH1-BamH1 de l'Herpes virus simplex, le traitement de restriction élimine le gène tk.

Le produit de ligation pTG156 comporte dans sa partie intéressante le gène de la rage sous le contrôle du promoteur de la thymidine kinase, le gène étant suivi du site de polyadénylation de SV40 (A).

L'introduction de l'intron de la globine est effectuée à partir du phage M13TG140 (voir ci-dessus) sous forme d'un fragment BglII/BamH1 qui est inséré dans le site BglII de pTG156 après délétion du fragment BhlII/BglII correspondant.

Cette délétion ayant supprimé le gène de la rage, celui-ci est réintroduit dans le site unique BglII de pTG156 sous forme d'un fragment BglII/BglII de pTG147. On obtient ainsi le plasmide pTG158.

La partie essentielle de ce plasmide devant être prélevée sous forme d'un fragment SalI/SalI (l'un des rares sites unique de pMOP) on introduit en amont du promoteur au niveau du site BamHI, un petit fragment provenant de M13TG120 et comportant un site SalI. M13TG120 est mis en digestion avec BglII et BamHI et pTG158 avec BamHI l'ensemble est lié pour donner pTG158 SAL.

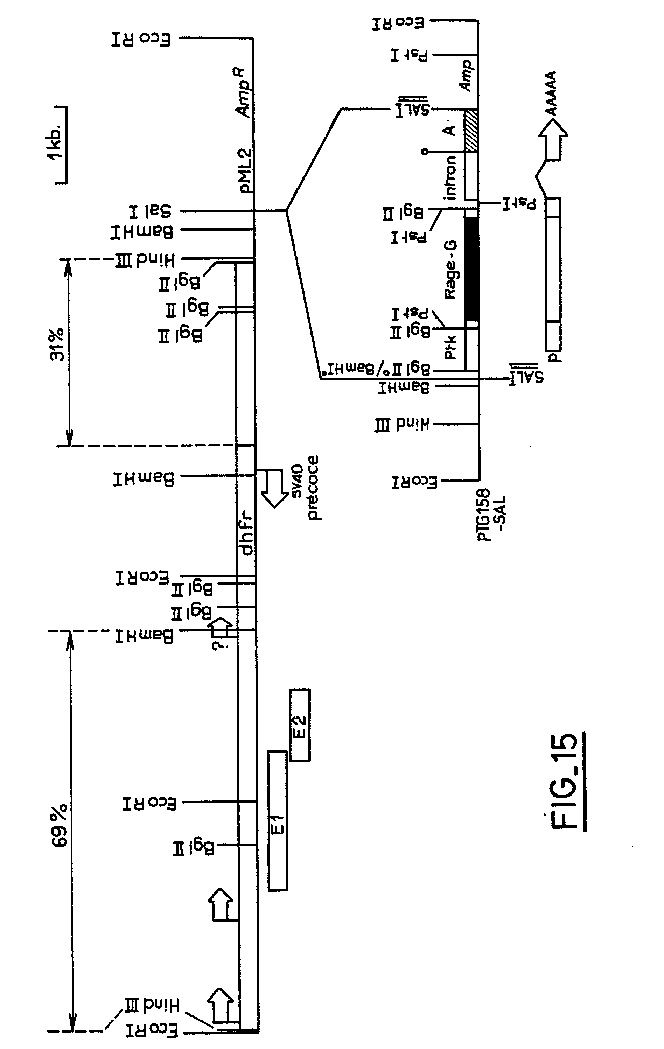

3) <u>Synthèse du vecteur</u> - figure 15

Par digestion de pTG158 et de pMOP avec SalI et ligation on obtient le plasmide pTG172 qui comporte, outre l'ensemble des éléments rappelés pour pMOP, le gène de la rage sous le contrôle du promoteur Ptk et terminé par l'intron de la $\beta$ globine et un site de polyadénylation.

Par transfection de cellules de mammifères ce vecteur produit une protéine antigénique de la rage, comme cela sera démontré ci-après.

**Exemple 5 : Transformation des cellules
3 T 3 NIHA (fibrolastes de souris)**

On utilise pour ces transformations le plasmide pMOP d'origine et le plasmide pTG172.

Les cellules sont mises en culture à raison de
$8.10^5$ cellules / boite de Petri de 10 cm de diamètre en
milieu Dulbecco + 10 % SVNN + antibiotique. (SVNN :
sérum de veau nouveau-né).

La transfection avec les vecteurs est effectuée
après 24 heures de culture par la technique de Wigler
et al. 1978.

Le milieu est changé 24 heures après la transfection. Puis on effectue un repiquage sur milieu sélectif contenant du méthotrexate.

Après 2 à 3 semaines on peut fixer les cellules en colonie et mettre en évidence les antigènes produits en utilisant l'immunofluorescence.

Des cellules de la lignée 3T3 ont été transfectées
par l'ADN purifié de

a) : plasmide pTG172-O (pMOP; vecteur BPV dhfr $^+$
sans insert additionnel).

b) : plasmide pTG172-rage (BPVTG172 ; vecteur
BPV dhfr $^+$ avec un insert additionnel qui comprend les différents éléments permettant l'expression de la glycoprotéine
de la rage).

Les colonies dérivant de cellules ayant incorporé
un plasmide et donc une résistance au méthotrexate (dhfr $^+$)
ont été fixées par traitement à l'acétone. L'expression de
la glycoprotéine rabique a été visualisée par traitements
succesifs avec un anticorps monoclonal (n° 509-6) dirigé
contre la glycoprotéine et un deuxième anticoprs fluorescent dirigé contre ce premier anticorps.

Agrandissement : environ 1.000 fois.

0140762

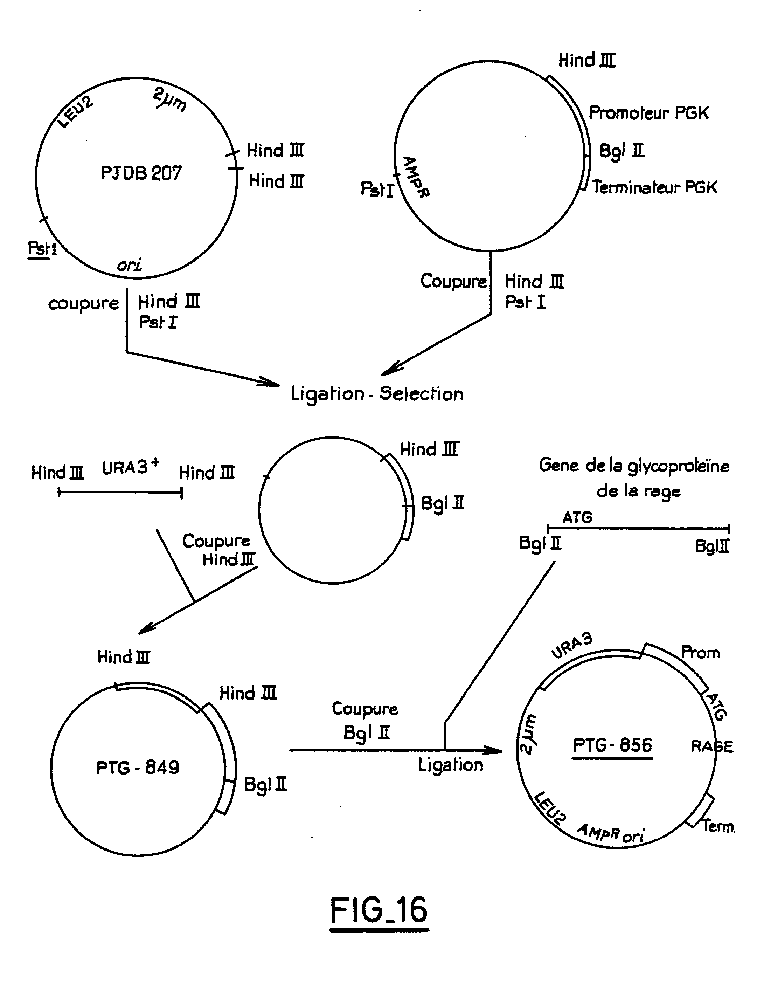

Exemple 6 : Construction du plasmide pTG 856 comprenant le gène de la glycoprotéine de la rage.(Figure 16).

On a isolé le gène PGK de levure à partir d'une banque de DNA chromosomique réalisée par intégration de fragments coupés au hasard (digestion partielle par Sau3A) au site BamHl d'un plasmide (pFL 1) capable de répliquer à la fois dans la levure et dans E. coli (Chevallier et al, 1980). La détection d'un fragment contenant le gène PGK a été réalisée par hybridatic in situ de colonies bactériennes, transformées par cette banque, avec une sonde spécifique (5'-TGTTTAGTTTAGTAGAACCTCGTGAAACTT-3') (Maniatis et al, 1982). La région 5' du gène PGK a été reclonée sous forme d'un fragment HindIII - SalI (2.3. kb) dans pBR 322.

Ce fragment comporte l'intégralité des séquences impliquées dans le contrôle de l'initiation de la transcription (promoteur) du gène PGK, plus le début de la séquence codante (Hitzeman et al, 1982).

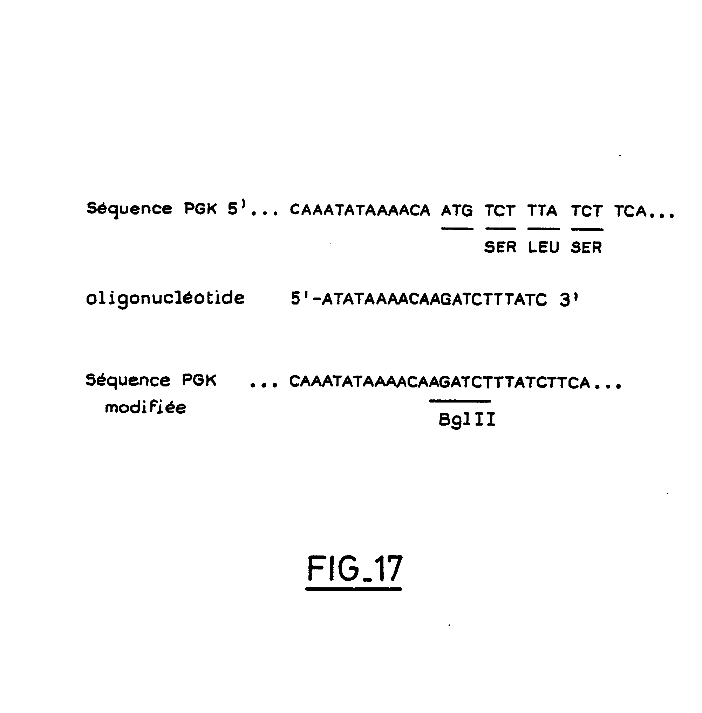

On a créé un site BglII détruisant l'ATG ouvrant la séquence codante du PGK, par mutagénèse invitro (Smith et Gillam,1979) (figure 17).

D'autre part, un fragment EcoRI - HindIII (0,5. kb) comprenant la fin (3') de la séquence codante et les signaux de terminaison transcriptionnelle du PGK a été traité à la polymérase Klenow et ligé au site PvuII unique du plasmide décrit au paragraphe précédent, de façon que le site EcoRI ainsi reconstitué soit en amont du site HindIII détruit. Ce nouveau fragment intégré comprend un site BglII localisé dans la partie codante du gène PGK. La délétion du fragment compris entre les deux sites BglII, celui nouvellement créé en aval du promoteur PGK et celui en amont du terminateur, laisse un site BglII unique où l'on peut cloner les gènes que l'on veut exprimer.

Le fragment HindIII - Pst 1 comprenant dans l'ordre la partie promotrice du gène PGK, le site BglII, la partie terminatrice du gène PGK,l'origine de réplication de pBR 322 et une partie de la séquence du gène Amp[r] conférant la résistance à l'ampicilline a été ligaturé avec le plus grand fragment résultant de la double digestion HindIII - Pst 1 du plasmide pJDB 207 de façon à reconstituer le gène Amp[r] (Beggs, 1981) (cf. figure 16). Le plasmide résultant (pTG 841) possède donc : le gène LEU2 de levure, le gène Amp$^r$ de E. coli, le gène REP3 du plasmide 2 µ de levure, l'origine de réplication du plasmide 2 µ de levure, l'origine de réplication de pBR 322, le promoteur et le terminateur du gène PGK de levure.

Un fragment HindIII de 1.1 kb comprenant le gène URA3 de levure (Brevet européen n° 78 3210) a été cloné au site HindIII de pTG 902, un dérivé de pBR 322 dépourvu des sites PstI et NdeI, pour former le plasmide pTG 802. Le DNA de ce plasmide a été linéarisé par coupure au site PstI unique — localisé dans la région 5' non codante du gène URA3 —, traité à la polymérase Klenow pour rendre franches les extrémité: libérées par la digestion PstI, et recircularisé par l'action de la DNA ligase du phage T4. Le fragment HindIII de 1.1 kb ainsi modifié (dépourvu de site PstI) a été intégré au site HindIII unique du plasmide pTG 841 de façon que le sens de transcription du gène URA3 soit divergent par rapport à celui du PGK (plasmide pTG 849). Le plasmide PTG 849 possède donc les mêmes caractéristiques que pTG 841, avec en plus la présence du gène URA3 modifié.

Le cDNA de la glycoprotéine de la rage a été isolé sous forme d'un fragment BglII comprenant l'intégralité de la séquence codante plus les extrémités polydGdC. Un site MstII est situé au début (5') de la séquence codante, l'enzyme coupant entre le septième et le huitième nucléotide suivant le A du ATG initiateur. Une séquence synthétique a été conçue pour substituer le fragment BglII - MstII dans la région 5' du gène, de façon à remplacer l'extrémité 5' polydGdC par une séquence plus proche des séquences non traduites des ARNs messager de levure (figure 17). Le fragment BglII ainsi modifié a été intégré au site BglII unique de pTG 849, de façon que le sens de transcription du gène de la glycoprotéine rabique soit le même que celui du PGK.

Exemple 7 : Expression de polypeptide de la glycoprotéine du virus de la rage dans la levure.

Le plasmide PTG856 est introduit dans la souche S.Cerevisiæ TGYlspl suivant la technique aux sels de lithium (Ito & coll 1983).

La même souche a été transformée par PTG849 qui est le même plasmide que PTG856 mais qui ne comporte pas le gène de la glycoprotéine. Ces souches ont été cultivées dans 25 ml de milieu sans uracile permettant de stabiliser le plasmide.

On ajoute de la cystéine radioactive $S^{35}$ pour marquer les protéines de la levure.

Les cellules sont broyées dans le tampon suivant :

250 µl : 25 mM Tris HCl pH 7,5
10 mM $Na_2$ EDTA pH 8.

250 µl : 1M.KCl
0,1 M Tris/HCl pH 8,8
2 % Triton X 100

5 µl : P.M.S.F. Phényl-méthyl-sulfunyl-fluoride

5 µl : "Soybean Trypsin inhibitor" 10 mg/m
Iodoacetamide 100 mM
N-éthylmaléimide 10 mM
1,10 - phenanthroline 10 mM

5 µl : DNAse I (0,5 mg/ml)

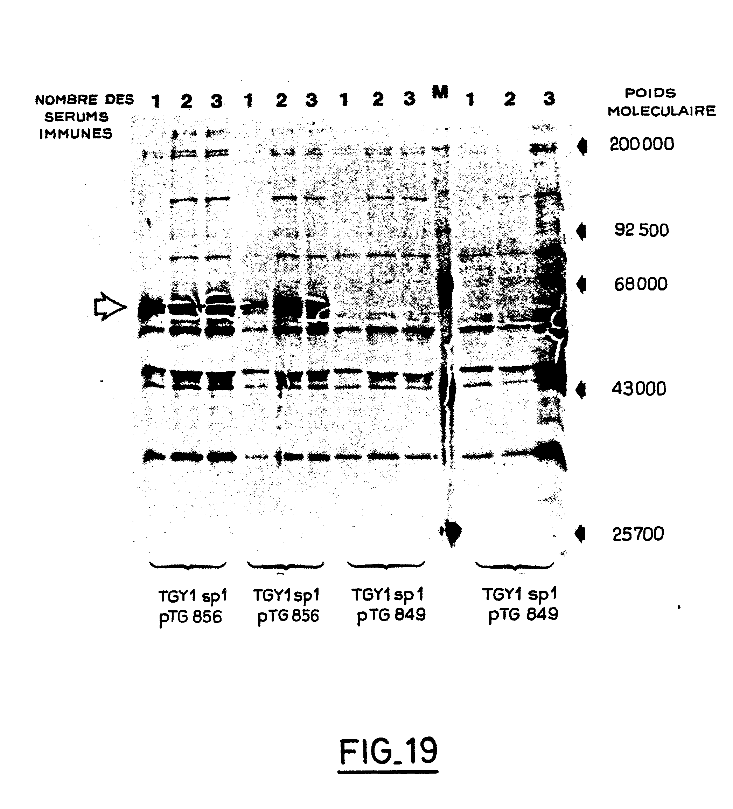

Les surnageants de ces broyats contenant les protéines radioactives sont incubés une nuit à 4° avec 5 µl de sérum de lapin. Deux sérums étaient dirigés contre la glycoprotéine (lots 2726 et 2527) et un contre le virus total (lot 2725). On ajoute 50 µl de protéine A- sépharose (Pharmacia CL-4 B). Après incubation, le culot est lavé 4 fois avec un tampon (0,5 M Kcl ; 10 mM Tris.Hcl pH 8,8 ; 0,5 % Triton X100) et trois fois avec un second tampon (10 mM Tris/Cl pH 8,8). Le culot est incubé 10 minutes à 100°C dans un tampon de dénaturation (62 mM Tris.HCl pH 6,8 ; 2 % SDS ; 5 % β-mercaptoéthanol ; 0,01 % bleu de bromophénol). L'extrait est alors analysé sur gel de polyacrylamide suivant la technique de Laemmli (1970). Après exposition du gel à un film photographique et développement, les différentes bandes correspondant aux protéines sont visibles (figure 19).

On peut en particulier voir dans les extraits de souches portant PTG 856 (a) et (b) une bande fortement marquée de poids moléculaire environ 65000 indiquée par une flèche qui est absente des extraits de souche portant PTG 849 (c) et (d). Ce poids moléculaire est celui attendu pour la glycoprotéine du virus de la rage.

Les souches suivantes ont été déposées le 30 septembre 1983 dans la Collection Nationale de cultures de microorganismes (CNCM) de l'Institut Pasteur

E.Coli 1106 / pTG 152 n° I 247
E.Coli 1106 / pTG 171 n° I 248
E.Coli 1106 / pTG 172 n° I 249
S.Cereviseae TGY1sp1 / pTG 856 n° I 250

Les plasmides pK14, pMOP et pSXBETA ont été aimablement communiqués par R. Breathnach.

EXEMPLE 8

Afin de tester si la glycoprotéine du virus de la rage exprimé dans la levure, détectée par immunoprécipité, était effectivement glycosylée, la glycoprotéine a été soumise à l'action de l'endo-β-N acétyglucosaminidase H in vitro. D'autre part, l'action de la tunicamycine in vivo sur la glycoprotéine exprimée dans la levure a également été examinée.

L'endo-β-N acétylglucosaminidase H clivera la liaison entre les résidus N acétylglucosamine du groupement N acétylchitibiose lié au résidu asparagine, lorsque trois conditions sont remplies :

- le groupement glycosylé doit être de nature neutre, c'est-à-dire constitué, en plus du N acétylchitibiose, uniquement de résidus mannoses (en ce qui concerne la partie sucre) ;

- il faut au minimum cinq résidus mannoses couplés au groupement N acétylchitibiose ;

- le groupement glycosylé doit être accessible à l'enzyme endo-β-N acétylglucosaminidase H ; si dans la conformation native de la protéine considérée, ceci n'est pas le cas, l'action enzymatique de l'endo H doit être effectuée dans ces conditions partiellement dénaturantes (SDS 0,5 %, β mercaptoéthanol 50 mM).

Protocole expérimental : Les cellules (ségrégeant TGY2 spl3b identique à TGY1 mais leu$_2$⁻ au lieu de his⁻ exprimant ou non la glycoprotéine de la rage, c'est-à-dire transformée par pTG 856 ou pTG849) dont les protéines ont été préalablement marquées à la méthionine [35] S (254 µCi à raison de 115 Ci/mmoles, marquage 10 minutes) sont récoltées en phase exponentielle de croissance (milieu YNBG X leucine 20 µg/mg) et resuspendus dans le tampon Tris-HCl 12,5 mM pH 7,5, EDTA 5 mM pH 8, KCl 0,5 M, Tris-HCl 50 mM pH 8,8 TRITON X-100 1 %, PMSF 2 mM additionné de 2,5 µg de DNAse I.

Après broyage à l'aide de billes de verre et légère centrifugation, 4 10⁶ cpm-correspondant à un aliquote du surnageant - sont engagés dans un immunoprécipité avec 5 µl de sérum polyclonal de lapin n° 215 dressé contre la glycoprotéine de la rage (à 4° pendant 16 h).

Après addition de 8 mg de protéine A sépharose et incubation d'une heure à température ambiante, le culot de protéine A sépharose obtenu après centrifugation est remis en suspension dans 200 µl de tampon SDS 0,5 % BSA 0,1 mg/ml, citrate de Na pH 5,5 0,27 M, β mercaptoéthanol 50 mM. Après chauffage à 100 ° C pendant 10 minutes, deux fractions, chacune de 100 µl, sont constituées. A la première est additioné 1 µl (50 nanog) d'endo-β-N acétylglucosaminidase H (NEN) ; les deux fractions sont ensuite incubées à 37°C pendant 16 heures.

Une dyalise extensive des deux fractions est effectuée contre du SDS 0,5 % en solution aqueuse ; ensuite, les concentrations finales des échantillons sont amenées à 2 % en SDS et à 5 % en β-mercaptoéthanol. Les fractions sont bouillies pendant 10 minutes pour être ensuite déposées sur gel de polyacrylamide 10 %.

L'analyse des résultats montre que l'endo-β-N acétylglucosaminidase H diminue le poids moléculaire de la sous-unité de la glycoprotéine rabique produite dans la levure d'environ 5000 Daltons (respectivement 63.000 sans endo H et 58.000 après action de l'endo H) ce qui correspond au poids moléculaire de la glycoprotéine mature exprimée dans E. coli).

Il a été démontré que, dans les conditions expérimentales utilisées, aucune activité protéolytique - résultant d'une contamination de l'endo H - n'est détectable.

La tunicamycine, mélange d'antibiotiques de structures apparentées, produite à partir de Streptomyces lysosuperificus inhibe la première étape de synthèse du précurseur $(glc)_2$ $(man)_9$ $(glcNAc)_2$-P-P-dolichyl.

Son action in vivo est bien documentée et permet de mettre en évidence les précurseurs de glycoprotéines totalement dépourvus de sucres au niveau des résidus asparagines.

**Protocole expérimental :**

Les souches de levure (TGY2 sp13b exprimant ou non la glycoprotéine de la rage), en phase exponentielle de croissance sur YNBG + leucine 20 µg/ml, sont préincubées avec de la tunicamycine (15 µg/ml) pendant 30 minutes. Un test préliminaire a montré que cette concentration conduit à une absence totale de croissance sur ce milieu pour les souches utilisées.

Après cette préincubation, de la méthionine $^{35}$S (254 µCi à raison de 1115 Ci/mmoles) est additionnée au milieu et les cellules sont récoltées après 10 minutes de marquage. Elles sont resuspendues dans le tampon TRIS-Cl 12,5 mM pH 7,5, EDTA 5 mM pH 8, KCl 0,5 M, TRIS-Cl 50 mM pH 8,8, TRITON X-100 1 %, PMSF 2 mM additionné de 2,5 µg de DNAse I.

Après broyage à l'aide de billes de verre et légère centrifugation, 2 $10^6$ cmp - correspondant à un aliquote du surnageant - sont engagés dans un immunoprécipité avec 5 µl de sérum polyclonal de lapin n° 215 dressé contre la glycoprotéine native de la rage (à 4°C pendant 16 heures).

Après addition de 8 mg de protéine A sépharose et incubation d'une heure à température ambiante, le culot de protéine A sépharose, obtenu après centrifugation, est lavé 3 fois avec KCl 0,5 M TRIS-Cl pH 8,8 10 mM TRITON X-100, 0,5 % et 3 fois avec TRIS-Cl 10 mM pH 8,8. Le culot est enfin resuspendu dans 30 µl du mélange de dénaturation (SDS 10 %, β-mercaptoéthanol 25 %, glycérol 50 %, TRIS-CL pH 6,8 0,325 M), bouilli 10 minutes à 100°C, pour être ensuite déposé sur gel de polyacrylamide 10 %.

L'analyse des résultats montre que l'effet in vivo de la tunicamycine - exactement comme l'action de l'endo-β-N acétylglucosaminidase H in vitro - diminue le poids moléculaire de la sous-unité de la glycoprotéine rabique produite dans la levure d'environ 5000 Dalton.

La similitude des résultats, après-l'emploi de deux méthodologies tout à fait différentes - action de l'endo H in vitro et in vivo de la tunicamycine - montre clairement que la glycoprotéine du virus de la rage produite dans la levure est effectivement glycosylée.

## REFERENCES

* ANILIONIS, W.H. WUNNER & P.J. CURTIS, Nature, vol.294 pp 275-278 (1981)

* BEGGS J. (1981) In Genetics Engineering, vol.2, 175-203 Ed. R. WILLIAMSON, Academic Press

* CHEN et Coll. Nature vol.299, pp 529-534 (1982)

* CHEVALLIER M.R., BLOCH J.C., LACROUTE F., Gène 11, 11-19 (1979)

* DAGERT M. et ERHLICH S.D., Gène, 23-28 (1979)

* FINK et Coll. Natl. Sci. USA, 75, (1978)

* GILLAM S. & M. SMITH, Gène 8, 81-97 (1979)

* GRIFFIN B.E., DNA tumor viruses (1981)

* HARDISON et Coll. Cell, Vol. 18, 1285-1297 (1979)

* R.A. HITZEMAN, F.E. HAGIE, J.S. HAYFLICK, CHEN, P.H. SEEBURG, R.DERYNCK, Nucleic Acids Research 10, 7791-7808

* HOOPES et Coll., Nucleic Acid Research 9, 5493-5504 (1981)

* ISK-HOROWITZ et Coll., Nucleic Acids Research 9, 2989-2998 (1981)

* M.ITO, Y.FUKUDA, K.MURATA, A.KIMURA (1983) J.Bacteriol, 153, 1 p.163-168

* LAEMMLI UK, Nature 227, 680-685 (1970)

* LATHE et Coll., Genetic Engineering Ed. Bob Williamson vol.4 p.1-51 (1983)

* LUSKY et Coll., Nature 293, 79-81 (1981)

* MANIATIS T., E.F. FRITSCH, J.SAMBROOK (1982) in molecular Cloning, p.309 Ed. C.S.H. laboratory

* MIYANOHACA, A. TOH-E, C. NAZAKI, F.NAMADA, N.OKTOMO & K.MATSUBARA (1983) Proc. Nat. Acad. Sci.USA 80, 1.5

* ORR. WEANER T.L., SZOSTAK J.W, ROTHSTEIN R.J.(1981) Proc. Natl. Acad. Sci. (USA) 78 (10) 6354-6358

0140762

* SOBERON et Coll., Gène 9, 287-305 (1980)
* VALENSUELA P., A. MEDINA, W.RUTTER, G.AMMERER &
   B.D. HALL , Nature 298, 347-350 (1982)
* WIGLER et Coll., Cell, vol.14, 725-731 (1978)

## REVENDICATIONS

1 - Vecteur d'expression d'une protéine antigénique de la rage dans les cellules eucaryotes, caractérisé en ce qu'il comporte au moins :

- une séquence d'ADN(1) codant pour ladite protéine antigénique de la rage, et
- les éléments d'expression de cette séquence dans une cellule eucaryote.

2 - Vecteur selon la revendication 1, caractérisé en ce qu'il s'agit d'un vecteur autonome comportant au moins une origine de réplication dans les cellules eucaryotes.

3 - Vecteur selon l'une des revendications 1 et 2, caractérisé en ce que la séquence d'ADN(1) est comprise entre les sites PstI-PstI.

4 - Vecteur selon l'une des revendications 2 et 3, caractérisé en ce qu'il s'agit d'un vecteur autonome comportant au moins une origine de réplication de virus.

5 - Vecteur selon la revendication 4, caractérisé en ce que l'origine de réplication est celle du virus SV40 ou du BPV.

6 - Vecteur d'expression selon l'une des revendications 1 à 5, caractérisé en ce que les éléments d'expression proviennent de l'ADN d'un virus.

7 - Vecteur d'expression selon la revendication 6, caractérisé en ce que les éléments d'expression comportent au moins tout ou partie d'un promoteur du virus SV40.

8 - Vecteur d'expression selon la revendication 6, caractérisé en ce que les éléments d'expression comportent au moins tout ou partie du promoteur de la thymidine kinase du virus Herpès simplex.

9 - Vecteur d'expression selon les revendications 1 à 8, caractérisé en ce qu'il comporte en outre un gène marqueur s'exprimant dans les cellules eucaryotes.

10 - Vecteur d'expression selon les revendications 1 à 9, caractérisé en ce que la séquence d'ADN(1) est suivi d'un intron.

11 - Vecteur d'expression selon les revendications 1 à 10, caractérisé en ce que la séquence d'ADN(1) est suivi d'un site de polyadémylation.

12 - Vecteur d'expression selon la revendication 6, caractérisé en ce que le vecteur d'expression est constitué de deux vecteurs complémentaires comportant chacun une origine de réplication de SV40, l'un des vecteurs porte la séquence d'ADN(1) sous le contrôle d'un promoteur de SV40 et une partie du génome de SV40, l'autre vecteur comporte la partie complémentaire dudit génome.

13 - Vecteur d'expression selon la revendication 12, caractérisé en ce que l'un des vecteurs complémentaires comporte au moins outre l'origine de réplication de SV40, l'ensemble de la région des gènes tardifs de SV40, le vecteur d'expression proprement-dit comporte au moins outre l'origine de réplication de SV40 et la séquence d'ADN(1) l'ensemble de la région des gènes précoces de SV40 et les éléments assurant l'expression du gène d'ADN(1).

14 - Vecteur d'expression selon la revendication 12, caractérisé en ce que les vecteurs utilisés sont le vecteur SVTG151 et le vecteur complémentaire pTG152, ou bien le vecteur SVTG160 et le vecteur complémentaire pTG152.

15 - Vecteur d'expression selon la revendication 12, caractérisé en ce que les vecteurs utilisés sont le vecteur SVTG171 et le vecteur pTG152.

16 - Vecteur d'expression selon la revendication 4, caractérisé en ce qu'il comporte tout ou partie du génome du BPV et une séquence comportant dans l'ordre tout ou partie d'un promoteur de virus, la séquence d'ADN(1), un intron et un site de polyadénylation.

17 - Vecteur selon la revendication 16, caractérisé en ce que le promoteur est un fragment du promoteur du gène de la thymidine kinase de l'Herpès simple , l'intron est l'intron d'une $\beta$ globine, le site de polyadénylation est le site de polyadénylation précoce de SV40.

18 - Vecteur selon la revendication 1, caractérisé en ce qu'il est le vecteur pTG172.

19 - Vecteur selon l'une des revendications 2 et 3, caractérisé en ce qu'il s'agit d'un plasmide comportant une origine de réplication dans les levures.

20 - Vecteur selon la revendication 19, caractérisé en ce qu'il s'agit de l'origine de réplication du plasmide 2 $\mu$.

21 - Vecteur selon les revendications 1 à 3, et 19 et 20, caractérisé en ce que les éléments d'expression proviennent d'une levure.

22 - Vecteur selon la revendication 21, caractérisé en ce que les éléments d'expression sont au moins constitués du promoteur de URA3 ou du promoteur de la phosphoglycerate kinase.

23 - Vecteur selon l'une des revendications 21 et 22, caractérisé en ce que les éléments d'expression comportent un terminateur du gène URA3 ou de la phosphoglycerate kinase.

24 - Vecteur selon les revendications 19 à 23, caractérisé en ce qu'il comporte un gène de marquage.

25 - Vecteur selon les revendications 19 à 24, caractérisé en ce qu'il s'agit du plasmide pTG856.

26 - Vecteur selon les revendications 1 à 25, caractérisé en ce qu'il comporte une origine de réplication dans les bactéries et un gène de résistance à un antibiotique s'exprimant dans les bactéries.

27 - Procédé de préparation d'une protéine antigénique de la rage, caractérisé en ce qu'on a cultivé des cellules transfectées selon la revendication 26 et que l'on isole la protéine.

28 - Protéine antigénique de la rage obtenue par la mise en oeuvre du procédé selon la revendication 27.

29 - Protéine antigénique de la rage selon la revendication 28, caractérisée en ce qu'il s'agit d'une protéine glycosylée par une levure.

30 - Protéine selon la revendication 29 caractérisée en ce que la glycosylation a été effectuée par une souche de S. cerevisiae transformée par un plasmide selon l'une des revendications du brevet principal.

31 - Protéine selon les revendications 29 et 30, caractérisée en ce qu'elle est glycosylées sur les résidus asparagine 204 et 319.

32 - Vaccin ou composition curative, caractérisée en ce qu'elle comporte à titre de principe actif au moins un protéine selon l'une des revendications 28 à 31.

GRABPST.DAT   MOD.

PstI                                  -19 1                                        61                                          1

CTG CAG GGG GGG GGG GGG GGA GGA AAG ATG GTT CCT CAG GCT CTC CTG TTT GTA CCC CTT CTG GTT TTT CCA TTG TGT TTT GGG AAA TTC
                                        Met Val Pro Gln Ala Leu Leu Phe Val Pro Leu Leu Val Phe Pro Leu Cys Phe Gly Lys Phe

91                        HindIII 121        HgiJII                              PvuII
CCT ATT TAC ACG ATA CTA GAC AAG CTT GGT CCC TGG AGC CCG ATT GAC ATA CAT CAC CTC AGC TGC CCA AAC AAT TTG GTA GTG GAG GAC
Pro Ile Tyr Thr Ile Leu Asp Lys Leu Gly Pro Trp Ser Pro Ile Asp Ile His His Leu Ser Cys Pro Asn Asn Leu Val Val Glu Asp

181                                          211                                      241
GAA GGA TGC ACC AAC CTG TCA GGG TTC TCC TAC ATG GAA CTT AAA GTT GGA TAC ATC TTA GCC ATA AAA ATG AAC GGG TTC ACT TGC ACA
Glu Gly Cys Thr Asn Leu Ser Gly Phe Ser Tyr Met Glu Leu Lys Val Gly Tyr Ile Leu Ala Ile Lys Met Asn Gly Phe Thr Cys Thr

271                                          301                                      331
GGC GTT GTG ACG GAG GCT GAA ACC TAC ACT AAC TTC GTT GGT TAT GTC ACA ACC ACG TTC AAA AGA AAG CAT TTC CGC CCA ACA CCA GAT
Gly Val Val Thr Glu Ala Glu Thr Tyr Thr Asn Phe Val Gly Tyr Val Thr Thr Thr Phe Lys Arg Lys His Phe Arg Pro Thr Pro Asp

AvaIII                              HpaII BstEII                          421
GCA TGT AGA GCC GCG TAC AAC TGG AAG ATG GCC GGT GAC CCC AGA TAT GAA GAG TCT CTA CAC AAT CCG TAC CCT GAC TAC CGC TGG CTT
Ala Cys Arg Ala Ala Tyr Asn Trp Lys Met Ala Gly Asp Pro Arg Tyr Glu Glu Ser Leu His Asn Pro Tyr Pro Asp Tyr Arg Trp Leu

AsuII                                    481                                      511                        XhoI AvaI
CGA ACT GTA AAA ACC ACC AAG GAG TCT CTC GTT ATC ATA TCT CCA AGT GTA GCA GAT TTG GAC CCA TAT GAC AGA TCC CTT CAC TCG AGG
Arg Thr Val Lys Thr Thr Lys Glu Ser Leu Val Ile Ile Ser Pro Ser Val Ala Asp Leu Asp Pro Tyr Asp Arg Ser Leu His Ser Arg

541                                      571                                      601                        AvaI
GTC TTC CCT AGC GGG AAG TGC TCA GGA GTA GCG GTG TCT TCT ACC TAC TGC TCC ACT AAC CAC GAT TAC ACC ATT TGG ATG CCC GAG AAT
Val Phe Pro Ser Gly Lys Cys Ser Gly Val Ala Val Ser Ser Thr Tyr Cys Ser Thr Asn His Asp Tyr Thr Ile Trp Met Pro Glu Asn

631                                      661                                      691
CCG AGA CTA GGG ATG TCT TGT GAC ATT TTT ACC AAT AGT AGA GGG AAG AGA GCA TCC AAA GGG AGT GAG ACT TGC GGC TTT GTA GAT GAA
Pro Arg Leu Gly Met Ser Cys Asp Ile Phe Thr Asn Ser Arg Gly Lys Arg Ala Ser Lys Gly Ser Glu Thr Cys Gly Phe Val Asp Glu

StuI HaeI          AhaIII    SphI                                          781                        NruI
AGA GGC CTA TAT AAG TCT TTA AAA GGA GCA TGC AAA CTC AAG TTA TGT GGA GTT CTA GGA CTT AGA CTT ATG GAT GGA ACA TGG GTC GCG
Arg Gly Leu Tyr Lys Ser Leu Lys Gly Ala Cys Lys Leu Lys Leu Cys Gly Val Leu Gly Leu Arg Leu Met Asp Gly Thr Trp Val Ala

ATG CAA ACA TCA AAT GAA ACC AAA TGG TGC CCT CCC GAT CAG TTG GTG AAC CTG CAC GAC TTT CGC TCA GAC GAA ATT GAG CAC CTT GTT
Met Gln Thr Ser Asn Glu Thr Lys Trp Cys Pro Pro Asp Gln Leu Val Asn Leu His Asp Phe Arg Ser Asp Glu Ile Glu His Leu Val

BgaI

AcyI

GTA GAG GAG TTG GTC AGG AAG AGA GAG GAG TGT CTG GAT GCA CTA GAG TCC ATC ATG ACA ACC AAG TCA GTG AGT TTC AGA CGT CTC AGT
Val Glu Glu Leu Val Arg Lys Arg Glu Glu Cys Leu Asp Ala Leu Glu Ser Ile Met Thr Thr Lys Ser Val Ser Phe Arg Arg Leu Ser

CAT TTA AGA AAA CTT GTC CCT GGG TTT GGA AAA GCA TAT ACC ATA TTC AAC AAG AGC TTG ATG GAA GCC GAT GCT CAC TAC AAG TCA GTC
His Leu Arg Lys Leu Val Pro Gly Phe Gly Lys Ala Tyr Thr Ile Phe Asn Lys Thr Leu Met Glu Ala Asp Ala His Tyr Lys Ser Val

AGA ACT TGG AAT GAG ATC CTC CCT TCA AAA GGG TGT TTA AGA GTT GGG GGG AGG TGT CAT CCT CAT GTG AAC GGG GTG TTT TTC AAT GGT
Arg Thr Trp Asn Glu Ile Leu Pro Ser Lys Gly Cys Leu Arg Val Gly Gly Arg Cys His Pro His Val Asn Gly Val Phe Phe Asn Gly

ATA ATA TTA GGA CCT GAC GGC AAT GTC TTA ATC CCA GAG ATG CAA TCA TCC CTC CTC CAG CAA CAT ATG GAG TTG TTG GAA TCC TCG GTT
Ile Ile Leu Gly Pro Asp Gly Asn Val Leu Ile Pro Glu Met Gln Ser Ser Leu Leu Gln Gln His Met Glu Leu Leu Glu Ser Ser Val

AccI

ATC CCC CTT GTG CAC CCC CTG GCA GAC CCG TCT ACC GTT TTC AAG GAC GGT GAC GAG GCT GAG GAT TTT GTT GAA GTT CAC CTT CCC GAT
Ile Pro Leu Val His Pro Leu Ala Asp Pro Ser Thr Val Phe Lys Asp Gly Asp Glu Ala Glu Asp Phe Val Glu Val His Leu Pro Asp

HindII

ApaI HgiJII

GTG CAC AAT CAG GTC TCA GGA GTT GAC TTG GGT CTC CCG AAC TGG GGG AAG TAT GTA TTA CTG AGT GCA GGG GCC CTG ACT GCC TTG ATG
Val His Asn Gln Val Ser Gly Val Asp Leu Gly Leu Pro Asn Trp Gly Lys Tyr Val Leu Leu Ser Ala Gly Ala Leu Thr Ala Leu Met

ClaI°

TTG ATA ATT TTC CTG ATG ACA TGT TGT AGA AGA GTC AAT CGA TCA GAA CCT ACG CAA CAC AAT CTC AGA GGG ACA GGG AGG GAG GTG TCA
Leu Ile Ile Phe Leu Met Thr Cys Cys Arg Arg Val Asn Arg Ser Glu Pro Thr Gln His Asn Leu Arg Gly Thr Gly Arg Glu Val Ser

505

GTC ACT CCC CAA AGC GGG AAG ATC ATA TCT TCA TGG GAA TCA CAC AAG AGT GGG GGT GAG ACC AGA CTG TGA GGA CTG GCC GTC CTT TCA
Val Thr Pro Gln Ser Gly Lys Ile Ile Ser Ser Trp Glu Ser His Lys Ser Gly Gly Glu Thr Arg Leu ***

PstI

ACG ATC CAA GTC CTG AAG ATC ACC TCC CCT TGG GGG GTT CTT TTT AAA AAA AAA AAA AAA AAA AAA AAA ACC CCC CCC CCC CCC CTG CAG

1710

FIG. 1b

FIG.2

FIG_3

FIG. 4

FIG.5

FIG_6

FIG.7

$$\underline{CTG\,CAG} \text{—} G_{23} \text{—} AG\,G\,A\,A\,A\,G\,ATG\,GTT\;\underline{CCT}\;\underline{CAG}\,GCT \text{——}\quad \mathbf{A}$$

Met Val Pro Gln Ala (above CCT CAG GCT region)

$$\underset{Pst\,\mathrm{I}}{} \qquad \underset{\uparrow Mst\,\mathrm{II}}{}$$

$$^{5'}GATCTAATATGGTTCC^{3'}$$
$$_{3'}ATTATACCAAGGAGT_{5'} \qquad \mathbf{B}$$

$$\text{——}\underline{AGATC}TAATATGGTT\;\underline{CCT}\;\underline{CAG}\,GCT\text{——}\quad \mathbf{C}$$
$$\underset{Bgl\,\mathrm{II}}{} \qquad \underset{Mst\,\mathrm{II}}{}$$

## FIG.8

FIG_9

pSXBeta+

```
                        ccaatctacacacgggggtagggattacatagttcaggacttggg̲c̲a̲t̲a̲a̲a̲a̲l̲
        PvuII          ⟩Transcription
ggcagagcaggg̲c̲a̲g̲c̲t̲g̲ctgcttACACTTGCTTTTGACACAACTGTGTTTACTTGCAATCCCCCAAAACAGACAGA
    ValHisLeuSerSerGluGluLysSerAlaValThrAlaLeuTrpGlyLysValAlaAsnValGluGluValGlyGly
ATGGTGCATCTGTCCAGTGAGGAGAAGTCTGCGGTCACTGCCCTGTGGGGCAAGGTGAATGTGGAAGAAGTTGGTGGT
    GluAlaLeuGlyArg
GAGGCCCTGGGC̲A̲G̲GTTGGTATCCTTTTTACAGCACAACTTAATGAGACAGATAGAAACTGGTCTTGTAGAAACAGAG
                  INTRON I                              LeuLeuValValTyr
TAGTCGCCTGCTTTTCTGCCAGGTGCTGACTTCTCTCCCCTGGGCTGTTTTCATTTTCT̲C̲AGGCTGCTGGTTGTCTAC
ProTrpThrGlnArgPhePheGluSerPheGlyAspLeuSerSerAlaHisAlaValMetSerAsnProLysValLys
CCATGGACCCAGAGGTTCTTCGAGTCCTTTGGGGACCTGTCCTCTGCACATGCTGTTTATGAGCAATCCTAAGGTGAAG

AlaHisGlyLysLysValLeuAlaAlaPheSerGluGlyLeuAsnHisLeuAspAsnLeuLysGlyThrPheAlaLys
GCTCATGGCAAGAAGGTGCTGGCTGCCTTCAGTGAGGGTCTGAATCACCTGGACAACCTCAAAGGCACCTTTGCTAAG
                        BamHI
LeuSerGluLeuHisCysAspLysLeuHisValAspProGluAsnPheArg
CTGAGTGAACTGCACTGTGACAAGCTGCACGTGGATCCTGAGAACTTCAG̲G̲GTGAGTTTGGGGACCCTTGATTGTTCT

TTCTTTTTCGCTATTGTAAAATTCATGTTATATGGAGGGGGCAAAGTTTTCAGGGTGTTGTTTAGAATGGGAAGATGT

CCCTTGTATCACCATGGACCCTCATGATAATTTTGTTTCTTTCACTTTCTACTCTGTTGACAACCATTGTCTCCTCTT

ATTTTCTTTTCATTTTCTGTAACTTTTTCGTTAAACTTTAGCTTGCATTTGTAACGAATTTTTAAATTCACTTTTGTT
                    INTRON II
TATTTGTCAGATTGTAAGTACTTTCTCTAATCACTTTTTTTTCAAGGCAATCAGGGTATATTATATTGTACTTCAGCA

CAGTTTTAGAGAACAATTGTTATAATTAAATGATAAGGTAGAATATTTCTGCATATAAATTCTGGCTGGCGTGGAAAT

ATTCTTATTGGTAGAAACAACTACACCCTGGTCATCATCCTGCCTTTCTCTTTATGGTTACAATGATA⌐ACACTGTTT

GAGATGAGGATAAAATACTCTGAGTCCAAACCGGGCCCCTCTGCTAACCATGTTCATGCCTTCTTCTCTTTCCTACÂG
                                              EcoRI
LeuLeuGlyAsnValLeuValValValLeuSerHisHisPheGlyLysGluPheThrProGlnValGlnAlaAlaTyr
CTCCTGGGCAACGTGCTGGTTGTTGTGCTGTCTCATCATTTTGGCAAAGAATTCACTCCTCAGGTGCAGGCTGCCTAT

GlnLysValValAlaGluValAlaAlaAsnAlaLeuAlaHisLysTyrHis  BglII
CAGAAGGTGGTGGCTGGTGTGGCCAATGCCCTGGCTCACAAATACCAC̲T̲GAGATCTTTTTCCCTCTGCCAAAAATTAT

GGGGACATCATGAAGCCCCTTGAGCATCTGACTTCTGGCTAATAAAGGGAAATTTATTTTCATTGCaatagtgtgttgg

aattttttgtgtctctcactcgg
```

M13*tg*120

```
                            HindII
            BglII  SalI  BamHI  EcoRI
fMetThrMetIleThrGlnIleCysArgSerThrAspProGlyAsnSerLeuAla
ATGACCATGATTACGCAGATCTGCAGGTCGACGGATCCGGGGAATTCACTGGCC
            ̲P̲s̲t̲I
```

```
1   150  300  450  600  750  900  1050  1200  1350

█▬▬▬▬█▬▬▬▬▬▬▬▬▬▬▬▬▬▬▬▬▬▬▬▬▬▬▬▬▬█▬▬▬▬█
EXON 1  INTRON1  EXON 2      INTRON II        EXON 3
146bp    126bp   222bp        573bp           221bp
```

*Pvu*II +
*Bam*HI

*Hind*II +
*Bam*HI

```
                  PvuII°
        BglII    /HindII°              BamHI
                                        EcoRI

M13tg140 ————————□█▬▬▬ Intron I ▬▬█——————————
                  ⟨------- 489 bp -------⟩
                  PstI
```

# FIG_10

**FIG.11**

FIG_12

FIG_13

FIG_14

FIG_15

FIG_16

Séquence PGK 5'... CAAATATAAAACA ATG TCT TTA TCT TCA...

SER LEU SER

oligonucléotide    5'-ATATAAAACAAGATCTTTATC 3'

Séquence PGK    ... CAAATATAAAACAAGATCTTTATCTTCA...
  modifiée
                                BglII

# FIG.17

Extrémité 5' CDNA
glycoprotéine de
la rage

BglII

```
5'- AGATCT GCAG -- G₂₃ -- AGGAAAG ATG GTT CCT CAGG
```

Met Val Pro Gln

$5'- AGATCT\ GCAG\ --\ G_{23}\ --\ AGGAAAG\ ATG\ GTT\ CCT\ CAGG$

Pst1

Mst II

Adapteur
synthétique

```
5'-GATCTAATATGGTTCC-3'
   3'-ATTATACCAAGGAGT-5'
```

Extrémité modifiée
pour le clonage
dans le vecteur
d'expression

```
5'- AGATCT AATATGGTTCCTCAGGCT-3'
```

Fusion promoteur
du PGK - cDNA de
la glycoprotéine

Met Val

```
...CAAATATAAAACAAGATCTAATATG GGT...
```

## FIG_18

0140762

FIG_19

FIG. 20

Office européen
des brevets

**RAPPORT DE RECHERCHE EUROPEENNE**

0140762

Numéro de la demande

EP 84 40 1962

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | SCIENCE, volume 219, no. 4585, 11 février 1983; E. YELVERTON et al.: "Rabies virus glycoprotein analogs: biosynthesis in Escherichia coli", pages 614-620 * en entier * | 28 | C 12 N 15/00<br>C 12 P 21/02<br>A 61 K 39/205<br>C 07 K 15/14 |
| Y | IDEM | 1-7,11 -13,19 -24,26 -28 | |
| | --- | | |
| Y | FR-A-2 515 685 (THE WISTAR INSTITUTE) * en entier * | 1-7,11 -13,19 -24,26 -28 | |
| | --- | | DOMAINES TECHNIQUES RECHERCHES (Int. Cl.4) |
| Y | EP-A-0 073 656 (GENENTECH INC.) * en entier * | 1-7,11 ,26-28 | C 12 N<br>C 12 P<br>A 61 K<br>C 07 K |
| | --- | | |
| Y | EP-A-0 089 666 (GENENTECH) * page 8, lignes 10-33; page 20, lignes 1-37 * | 1-7,26 -28 | |
| | ---     -/- | | |

Le présent rapport de recherche a été établi pour toutes les revendications

| Lieu de la recherche | Date d'achèvement de la recherche | Examinateur |
|---|---|---|
| LA HAYE | 09-01-1985 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503 03 82

Office européen
des brevets

**RAPPORT DE RECHERCHE EUROPEENNE**

0140762

Numéro de la demande

EP 84 40 1962

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | PROCEEDINGS OF THE NATIONAL ACADEMY OF SCIENCES OF THE USA, volume 79, no. 2, janvier 1982, (WASHINGTON, US); J.R. HARTMAN et al.: "Human influenza virus hemagglutinin is expressed in monkey cells using simian virus 40 vectors", pages 233-237 * en entier * | 1-7,12 ,13,26 -28 | |
| Y | NATURE, volume 277, 11 janvier 1979, R.C. MULLIGAN et al.: "Synthesis of rabbit beta-globin in cultured monkey kidney recombinant genome", pages 108-114 * en entier * | 1-7,26 -28 | |
| Y | EP-A-0 060 057 (BOARD OF REGENTS UNIVERSITY OF WASHINGTON) * en entier * | 1,2,3 19-24 26-28 | DOMAINES TECHNIQUES RECHERCHES (Int. Cl.4) |
| Y | EP-A-0 073 635 (A.J. KINGSMAN et al.) * en entier * | 1,2,3 19-24 26-28 | |
| | --- -/- | | |

Le présent rapport de recherche a été établi pour toutes les revendications

| Lieu de la recherche LA HAYE | Date d'achèvement de la recherche 09-01-1985 | Examinateur DESCAMPS J.A. |
|---|---|---|

Office européen
des brevets

**RAPPORT DE RECHERCHE EUROPEENNE**

0140762
Numéro de la demande

EP  84 40 1962

Page   3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOCHEMICAL AND BIOPHYSICAL RESEARCH COMMUNICATIONS, volume 103, no. 2, 30 novembre 1981, C.Y. LAI et al.: "Amino acid composition and terminal sequence analysis of the rabies virus glycoprotein: identification of the reading frame on the cDNA sequence", pages 536-542 * page 537, lignes 8-25 * | 28 | |
| P,X | EP-A-0 117 657  (GENENTECH INC.) * page 9, ligne 4 - page 11, ligne 6 * | 1-7,11 ,19,21 -23,27 ,28 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 09-01-1985 | DESCAMPS J.A. |